(19) Europäisches Patentamt / European Patent Office / Office européen des brevets

(11) EP 3 670 508 B1

(12) **EUROPEAN PATENT SPECIFICATION**

(45) Date of publication and mention of the grant of the patent:
**01.10.2025 Bulletin 2025/40**

(21) Application number: **19213557.2**

(22) Date of filing: **04.12.2019**

(51) International Patent Classification (IPC):
***C07D 403/14*** *(2006.01)* ***C07D 495/04*** *(2006.01)*
***C07D 491/04*** *(2006.01)* ***C07D 487/04*** *(2006.01)*
***H10K 85/60*** *(2023.01)* ***H10K 101/20*** *(2023.01)*
***C09K 11/06*** *(2006.01)*

(52) Cooperative Patent Classification (CPC):
**C07D 403/14; C07D 487/04; C07D 491/04; C07D 495/04; C09K 11/06; H10K 85/654; H10K 85/6572;** H10K 50/11; H10K 2101/20; Y02E 10/549

(54) **ORGANIC MOLECULES FOR USE IN OPTOELECTRONIC DEVICES**

ORGANISCHE MOLEKÜLE ZUR VERWENDUNG IN OPTOELEKTRONISCHEN VORRICHTUNGEN

MOLÉCULES ORGANIQUES DESTINÉES À ÊTRE UTILISÉES DANS DES DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 DE 102018133257**

(43) Date of publication of application:
**24.06.2020 Bulletin 2020/26**

(73) Proprietor: **Samsung Display Co., Ltd.**
**Gyeonggi-do 17113 (KR)**

(72) Inventors:
- **DANZ, Michael**
**76344 Eggenstein-Leopoldshafen (DE)**
- **DIGENNARO, Angela**
**69120 Heidelberg (DE)**
- **SZAFRANOWSKA, Barbara**
**76646 Bruchsal (DE)**
- **VELLAIYAPPAN, Arun Pandian**
**76131 Karlsruhe (DE)**

(74) Representative: **Dr. Weitzel & Partner**
**Patent- und Rechtsanwälte mbB**
**Friedenstrasse 10**
**89522 Heidenheim (DE)**

(56) References cited:
**EP-A1- 3 275 968** **KR-A- 20160 059 733**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 670 508 B1

## Description

The invention relates to organic light-emitting molecules and their use in organic light-emitting diodes (OLEDs) and in other optoelectronic devices.

## Prior art

EP 3 275 968 A1 discloses a thermally activated delayed fluorescent (TADF) material. For example the light-emitting material has the chemical formula 7:

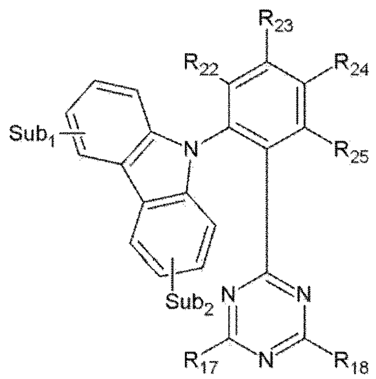

wherein $R_{17}$, $R_{18}$, $R_{22}$-$R_{25}$, Sub, and $Sub_2$ are defined as disclosed in EP 3 275 968 A1.

KR 2016 0059733 A describes a light emitting compound represented by chemical formula 1:

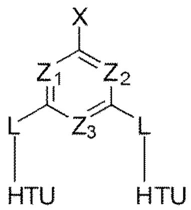

wherein $Z_1$-$Z_3$, X, L and HTU are defined as disclosed in KR 2016 0059733 A.

## Description

An object of the present invention is to provide molecules which are suitable for use in optoelectronic devices.

This object is achieved by the invention which provides a new class of organic molecules.

The organic molecules of the invention are preferably purely organic molecules, i.e. they do not contain any metal ions, which is in contrast to metal complexes known for use in optoelectronic devices. Therefore, according to the present invention, it is preferred that the organic molecules are free of metal atoms or metal ions. The pure organic molecules may, however, in concordance with modern era definitions include metalloids, in particular, B and Si or exceptionally Sn, Se and Ge.

According to the present invention, the organic molecules exhibit emission maxima in the blue, sky-blue or green spectral range. The organic molecules exhibit, in particular, emission maxima between 420 nm and 520 nm, between 440 nm and 495 nm, or between 450 nm and 470 nm. The photoluminescence quantum yields of the organic molecules according to the invention are, in particular, 20 % or more. The molecules according to the invention show, in particular, thermally activated delayed fluorescence (TADF). The use of the molecules according to the invention in an optoelectronic device, for example, in an organic light-emitting diode (OLED), leads to higher efficiencies of the device. Corresponding OLEDs have a higher stability than OLEDs with known emitter materials and comparable color.

The organic light-emitting molecules of the invention comprise or consist of a first chemical moiety comprising or consisting of a structure of formula Iaa,

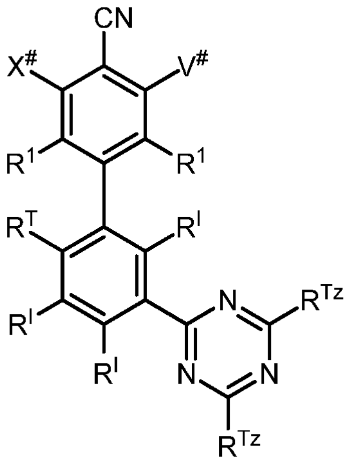

Formula Iaa

and

- three second chemical moieties, each independently from another comprising or consisting of a structure of formula II,

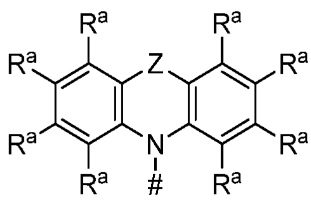

Formula II

wherein the first chemical moiety is linked to each of the three second chemical moieties via a single bond.
wherein
$X^{\#}$ is the binding site of a single bond linking the first chemical moiety to one of the three second chemical moieties,
$V^{\#}$ is the binding site of a single bond linking the first chemical moiety to one of the three second chemical moieties,
$R^T$ is the binding site of a single bond linking the first chemical moiety to one of the three second chemical moieties.
# represents the binding site of a single bond linking the second chemical moieties to the first chemical moiety;
Z is a direct bond;
$R^1$ is at each occurrence independently from another selected from the group consisting of: hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$.
$R^1$ is at each occurrence independently from another selected from the group consisting of: hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$.

$R^{Tz}$ is at each occurrence independently from another selected from the group consisting of hydrogen, deuterium,
$C_1$-$C_5$-alkyl,
wherein one or more hydrogen atoms are optionally substituted by deuterium;
$C_6$-$C_{18}$-aryl,
which is optionally substituted with one or more substituents $R^{6'}$; and
$R^{6'}$ is at each occurrence independently selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$, and
$N(C_3$-$C_{17}$-heteroaryl$)(C_6$-$C_{18}$-aryl$)$;
$R^a$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, $C\equiv C$, $Si(R^5)_2$,

$Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.
$R^5$ is at each occurrence independently from another selected from the group consisting of: hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$.
$R^6$ is at each occurrence independently from another selected from the group consisting of: hydrogen, deuterium, OPh, $CF_3$, CN, F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN, $CF_3$, or F;
$C_1$-$C_5$-thioalkoxy,
wherein optionally one or more hydrogen atoms are independently from each other substituted by deuterium, CN, $CF_3$, or F;
$C_2$-$C_5$-alkenyl,
wherein optionally one or more hydrogen atoms are independently from each other substituted by deuterium, CN, $CF_3$, or F;
$C_2$-$C_5$-alkynyl,
wherein optionally one or more hydrogen atoms are independently from each other substituted by deuterium, CN,

$CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$,
$N(C_3$-$C_{17}$-heteroaryl$)_2$; and
$N(C_3$-$C_{17}$-heteroaryl)($C_6$-$C_{18}$-aryl).

The substituents $R^a$ or $R^5$ independently from each other can optionally form a mono- or polycyclic, aliphatic, aromatic and/or benzo-fused ring system with one or more substituents $R^a$ or $R^5$.

In one embodiment, $R^1$ and $R^I$ is at each occurrence independently from another selected from the group consisting of hydrogen (H), methyl, mesityl, tolyl and phenyl. The term tolyl refers to 2-tolyl, 3-tolyl, and 4-tolyl.

In one embodiment, $R^1$ and $R^I$ is at each occurrence independently from another selected from the group consisting of hydrogen (H) and phenyl.

In one embodiment, $R^1$ and $R^I$ is at each occurrence hydrogen (H).

In one embodiment, V, X and $R^T$ is the binding site of a single bond linking the first chemical moiety to one of the three second chemical moieties.

In a further embodiment of the invention $R^{Tz}$ is independently from each other selected from the group consisting of H, methyl,
phenyl, which is optionally substituted with one or more substituents $R^6$.

In a further embodiment of the invention, $R^{Tz}$ is phenyl, which is optionally substituted with one or more substituents $R^6$.

In a further embodiment of the invention, $R^{Tz}$ is phenyl at each occurrence.

In a further embodiment of the invention, $R^a$ is at each occurrence independently from another selected from the group consisting of:

H,
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$.

In a further embodiment of the invention, $R^a$ is at each occurrence independently from another selected from the group consisting of: H,

Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from

the group consisting of Me, $^{i}Pr$, $^{t}Bu$, CN, $CF_3$, and Ph, and
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is at each occurrence independently from another selected from the group consisting of: H,

Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is H at each occurrence.

In a further embodiment of the invention, the three second chemical moieties each at each occurrence independently from another comprise or consist of a structure of formula IIb, a structure of formula IIb-2, a structure of formula IIb-3 or a structure of formula IIb-4:

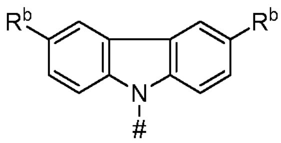
Formula IIb

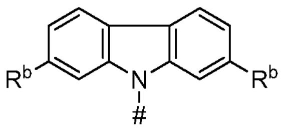
Formula IIb-2

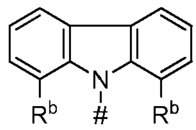
Formula IIb-3

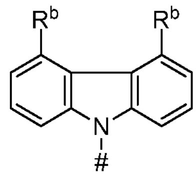
Formula IIb-4

wherein
$R^b$ is at each occurrence independently from another selected from the group consisting of:

H,
deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$,

$Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.

Apart from that, the aforementioned definitions apply.
In one additional embodiment of the invention, the three second chemical moieties each at each occurrence independently from another comprise or consist of a structure of formula IIc, a structure of formula IIc-2, a structure of formula IIc-3, or a structure of formula IIc-4:

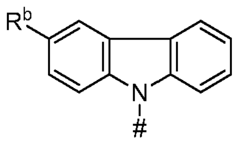
Formula IIc

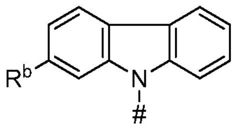
Formula IIc-2

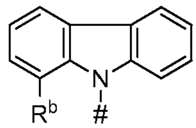
Formula IIc-3

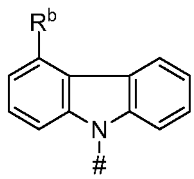
Formula IIc-4

wherein the aforementioned definitions apply.
In a further embodiment of the invention, $R^b$ is at each occurrence independently from another selected from the group consisting of:

H,
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph;
pyridinyl, which is optionally substituted with one or more substituents independently from each other selected from the group consisting of Me, $^i$Pr, $^t$Bu, CN, $CF_3$, and Ph;
pyrimidinyl, which is optionally substituted with one or more substituents independently from each other selected from the group consisting of Me, $^i$Pr, $^t$Bu, CN, $CF_3$, and Ph;
carbazolyl, which is optionally substituted with one or more substituents independently from each other selected from the group consisting of Me, $^i$Pr, $^t$Bu, CN, $CF_3$, and Ph;
triazinyl, which is optionally substituted with one or more substituents independently from each other selected from the group consisting of Me, $^i$Pr, $^t$Bu, CN, $CF_3$, and Ph; and
$N(Ph)_2$.

In a further embodiment of the invention, $R^b$ is at each occurrence independently from another selected from the group consisting of:

H,

Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph;
pyridinyl, which is optionally substituted with one or more substituents independently from each other selected from the group consisting of Me, $^{i}Pr$, $^{t}Bu$, CN, $CF_3$, and Ph;
carbazolyl, which is optionally substituted with one or more substituents independently from each other selected from the group consisting of Me, $^{i}Pr$, $^{t}Bu$, CN, $CF_3$, and Ph;
triazinyl, which is optionally substituted with one or more substituents independently from each other selected from the group consisting of Me, $^{i}Pr$, $^{t}Bu$, CN, $CF_3$, and Ph; and
$N(Ph)_2$.

In a further embodiment of the invention, $R^b$ is at each occurrence independently from another selected from the group consisting of:

H,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph;
pyridinyl, which is optionally substituted with one or more substituents independently from each other selected from the group consisting of Me, $^{i}Pr$, $^{t}Bu$, CN, $CF_3$, and Ph;
pyrimidinyl, which is optionally substituted with one or more substituents independently from each other selected from the group consisting of Me, $^{i}Pr$, $^{t}Bu$, CN, $CF_3$, and Ph; and
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^b$ is at each occurrence independently from another selected from the group consisting of:

H,
Me,
$^{t}Bu$,
Ph, which is optionally substituted with one or more substituents independently from each other selected from the group consisting of Me, $^{i}Pr$, $^{t}Bu$, CN, $CF_3$, and Ph; and
triazinyl, which is optionally substituted with one or more substituents independently from each other selected from the group consisting of Me, $^{i}Pr$, $^{t}Bu$, CN, $CF_3$, and Ph.

In the following, examples of the second chemical moiety are shown:

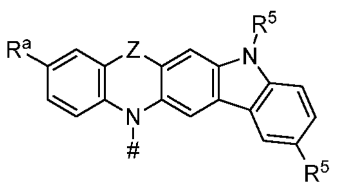
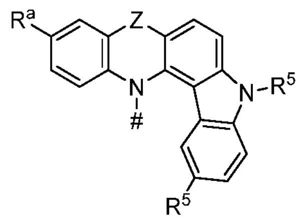
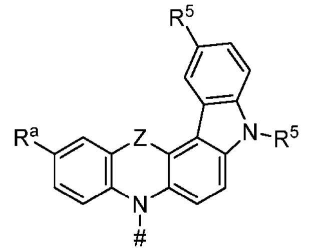
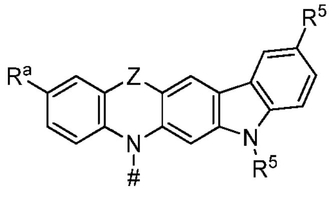
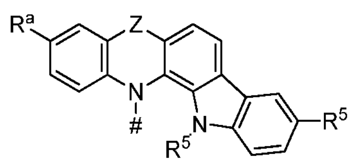
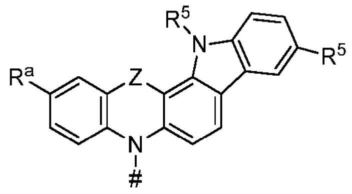
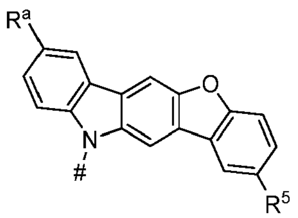
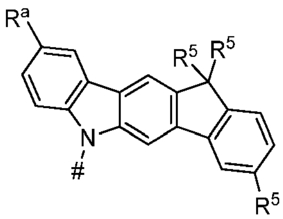
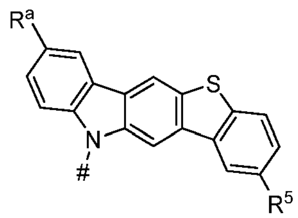
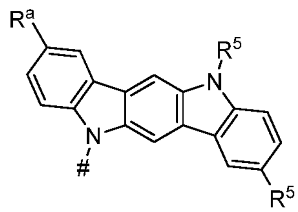
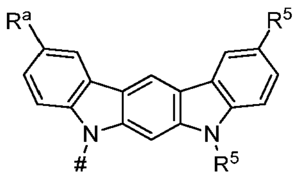
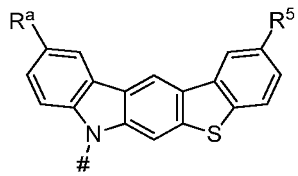
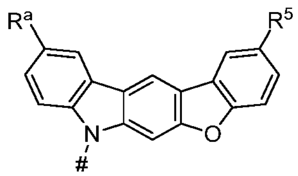
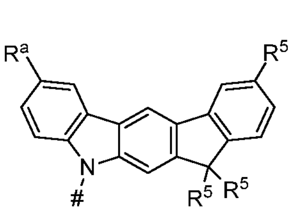
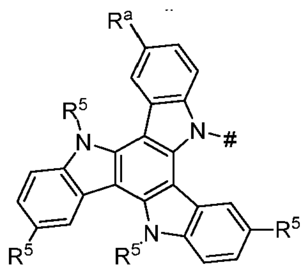
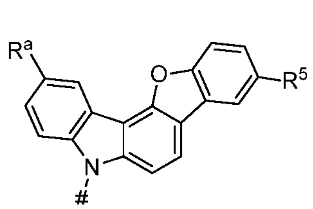
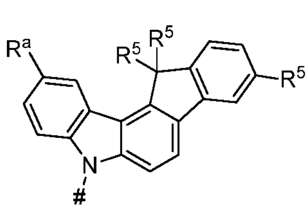
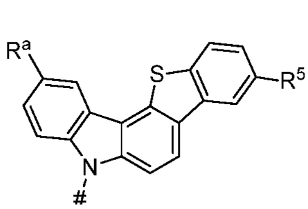

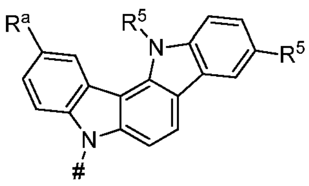
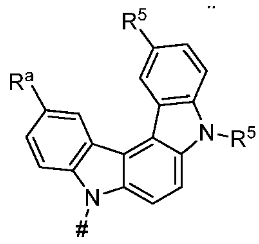
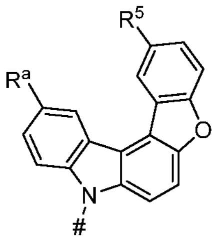
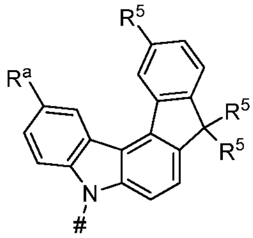
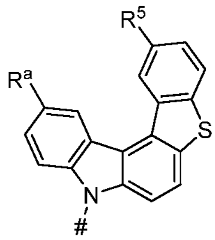
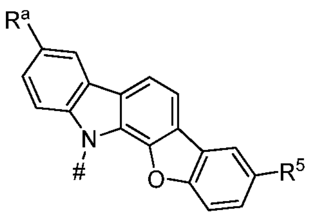
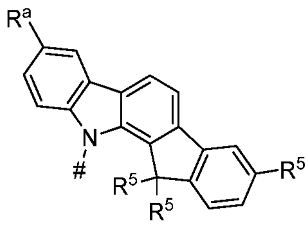
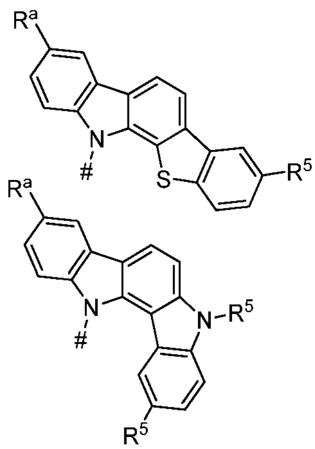
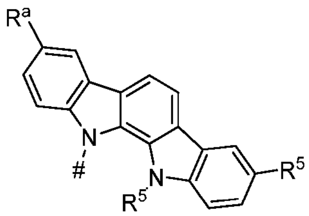
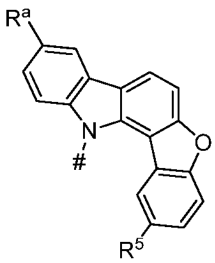
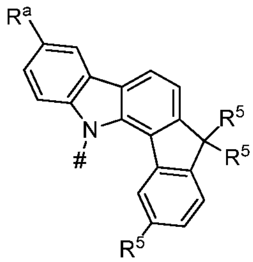
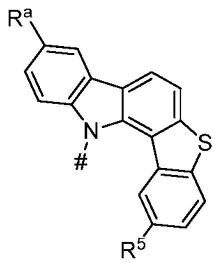

wherein for #, Z, $R^a$ and $R^5$, the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph), CN, $CF_3$, and diphenylamine ($NPh_2$).

In a further embodiment of the invention, the three second chemical moieties are identical at each occurrence.

In a further embodiment of the invention, at least two of the three second chemical moieties are identical.

In one embodiment of the invention, the organic molecules comprise or consist of a structure of formula III:

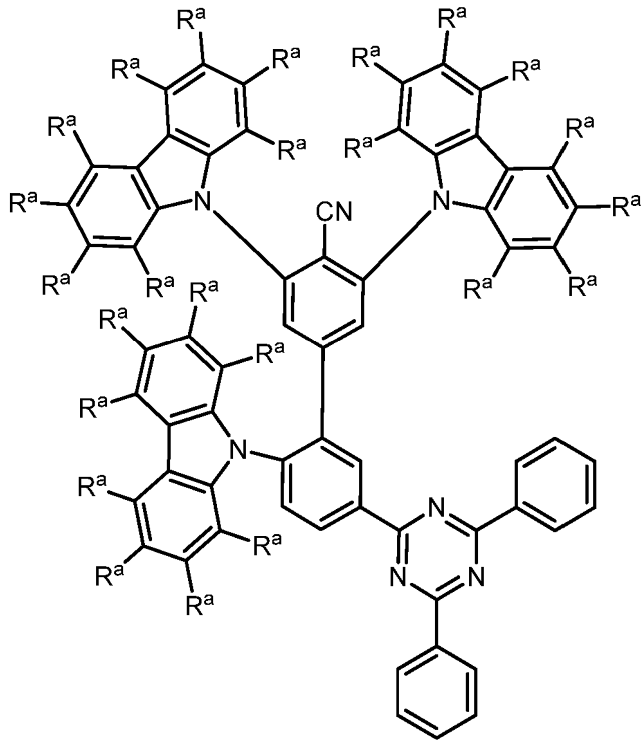

Formula III

wherein the aforementioned definitions apply.

In a further embodiment of the invention, the organic molecules comprise or consist of a structure of formula IIIa:

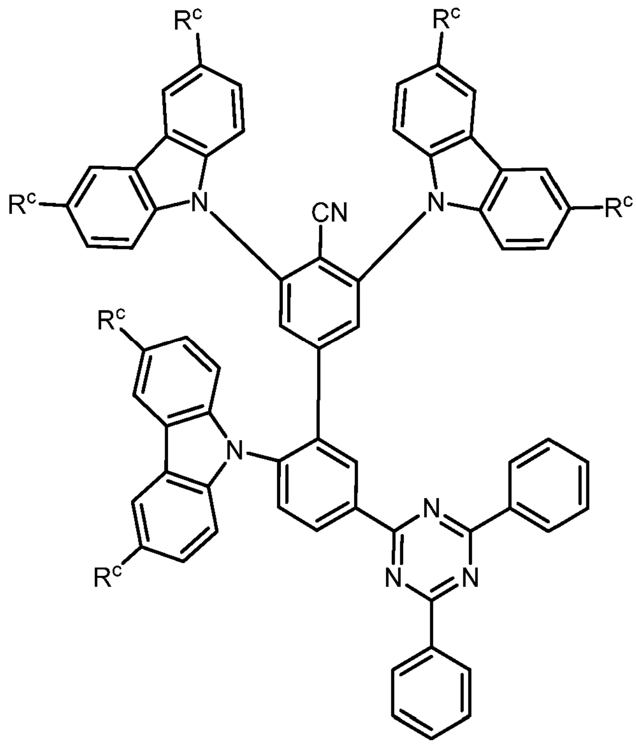

Formula IIIa

wherein

$R^c$ is at each occurrence independently from another selected from the group consisting of:

H,
Me,
$^{i}Pr$,
$^{t}Bu$,
Ph, which is optionally substituted with one or more substituents independently from each other selected from the group consisting of Me, $^{i}Pr$, $^{t}Bu$, CN, $CF_3$, and Ph;
pyridinyl, which is optionally substituted with one or more substituents independently from each other selected from the group consisting of Me, $^{i}Pr$, $^{t}Bu$, CN, $CF_3$, and Ph;
pyrimidinyl, which is optionally substituted with one or more substituents independently from each other selected from the group consisting of Me, $^{i}Pr$, $^{t}Bu$, CN, $CF_3$, and Ph;
carbazolyl, which is optionally substituted with one or more substituents independently from each other selected from the group consisting of Me, $^{i}Pr$, $^{t}Bu$, CN, $CF_3$, and Ph;
triazinyl, which is optionally substituted with one or more substituents independently from each other selected from the group consisting of Me, $^{i}Pr$, $^{t}Bu$, CN, $CF_3$, and Ph;
and $N(Ph)_2$.

In a further embodiment of the invention, the organic molecules comprise or consist of a structure formula IIIb:

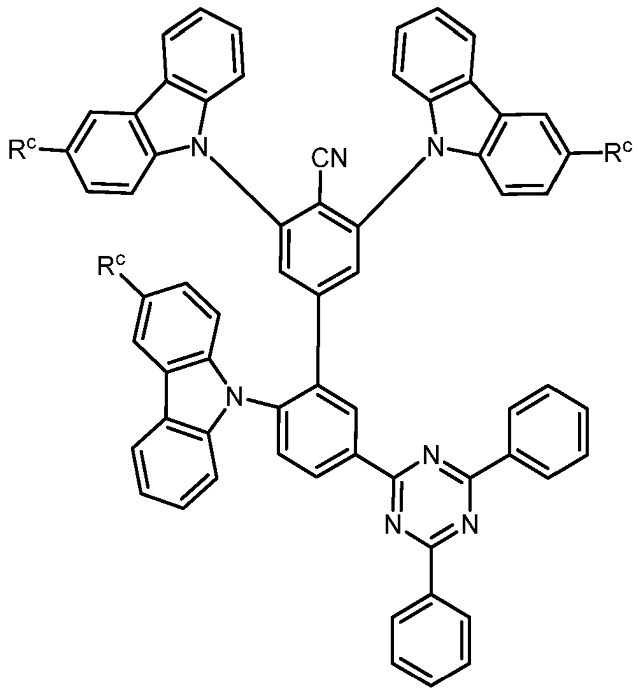

Formula IIIb

wherein the aforementioned definitions apply.

In one embodiment of the invention, $R^c$ is at each occurrence independently from another selected from the group consisting of:

H,
Me,
$^{i}Pr$,
$^{t}Bu$,
Ph, which is optionally substituted with one or more substituents independently from each other selected from the group consisting of Me, $^{i}Pr$, $^{t}Bu$, CN, $CF_3$ and Ph; and
triazinyl, which is optionally substituted with one or more substituents independently from each other selected from the group consisting of Me, $^{i}Pr$, $^{t}Bu$, CN, $CF_3$ and Ph.

As used throughout the present application, the terms "aryl" and "aromatic" may be understood in the broadest sense as any mono-, bi- or polycyclic aromatic moieties. Accordingly, an aryl group contains 6 to 60 aromatic ring atoms,

and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom.

The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout, the term "aryl group" or "heteroaryl group" comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout, the term "cyclic group" may be understood in the broadest sense as any mono-, bi- or polycyclic moieties.

As used throughout, the term biphenyl as a substituent may be understood in the broadest sense as ortho-biphenyl, meta-biphenyl, or para-biphenyl, wherein ortho, meta and para is defined in regard to the binding site to another chemical moiety.

As used throughout, the term "alkyl group" may be understood in the broadest sense as any linear, branched, or cyclic alkyl substituent. In particular, the term alkyl comprises the substituents methyl (Me), ethyl (Et), n-propyl ($^n$Pr), i-propyl ($^i$Pr), cyclopropyl, n-butyl ($^n$Bu), i-butyl ($^i$Bu), s-butyl ($^s$Bu), t-butyl ($^t$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout, the term "alkenyl" comprises linear, branched, and cyclic alkenyl substituents. The term alkenyl group, for example, comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout, the term "alkynyl" comprises linear, branched, and cyclic alkynyl substituents. The term "alkynyl group", for example, comprises ethynyl, propynyl, butynyl, pentynyl, hexynyl, heptynyl or octynyl.

As used throughout, the term "alkoxy" comprises linear, branched, and cyclic alkoxy substituents. The term "alkoxy group", for example, comprises methoxy, ethoxy, n-propoxy, i-propoxy, n-butoxy, i-butoxy, s-butoxy, t-butoxy and 2-methylbutoxy.

As used throughout, the term "thioalkoxy" comprises linear, branched, and cyclic thioalkoxy substituents, in which the O of the exemplarily alkoxy groups is replaced by S.

As used throughout, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more

than 150 μs, of not more than 100 μs, in particular of not more than 50 μs, more preferably of not more than 10 μs or not more than 7 μs in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In particular, the organic molecules according to the invention have an emission peak in the visible or nearest ultraviolet range, i.e., in the range of a wavelength of from 380 to 800 nm, with a full width at half maximum of less than 0.48 eV, preferably less than 0.45 eV, more preferably less than 0.42 eV, even more preferably less than 0.40 eV or even less than 0.39 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

In a further embodiment of the invention, the organic molecules according to the invention have a PLQY in % of more than 60%, in particular more than 64%, preferably more than 70%, more preferably of more than 75% or even more than 79%.

In a further embodiment of the invention, the organic molecules according to the invention have a PLQY in % of more than 75% or even more than 79%.

Orbital and excited state energies can be determined either by means of experimental methods or by calculations employing quantum-chemical methods, in particular, density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10 % by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10 % by weight of emitter. Both for host and emitter compounds, the energy of the first excited singlet state S1 is determined from the onset of the emission spectrum, if not otherwise stated measured in a film of PMMA with 10 % by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a method for preparing organic molecules (with an optional subsequent reaction) according to the invention:

Hal = Cl, Br or I

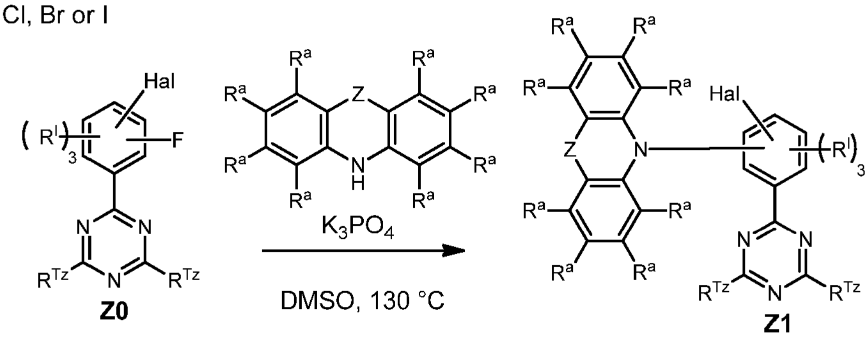


Z0 Z1

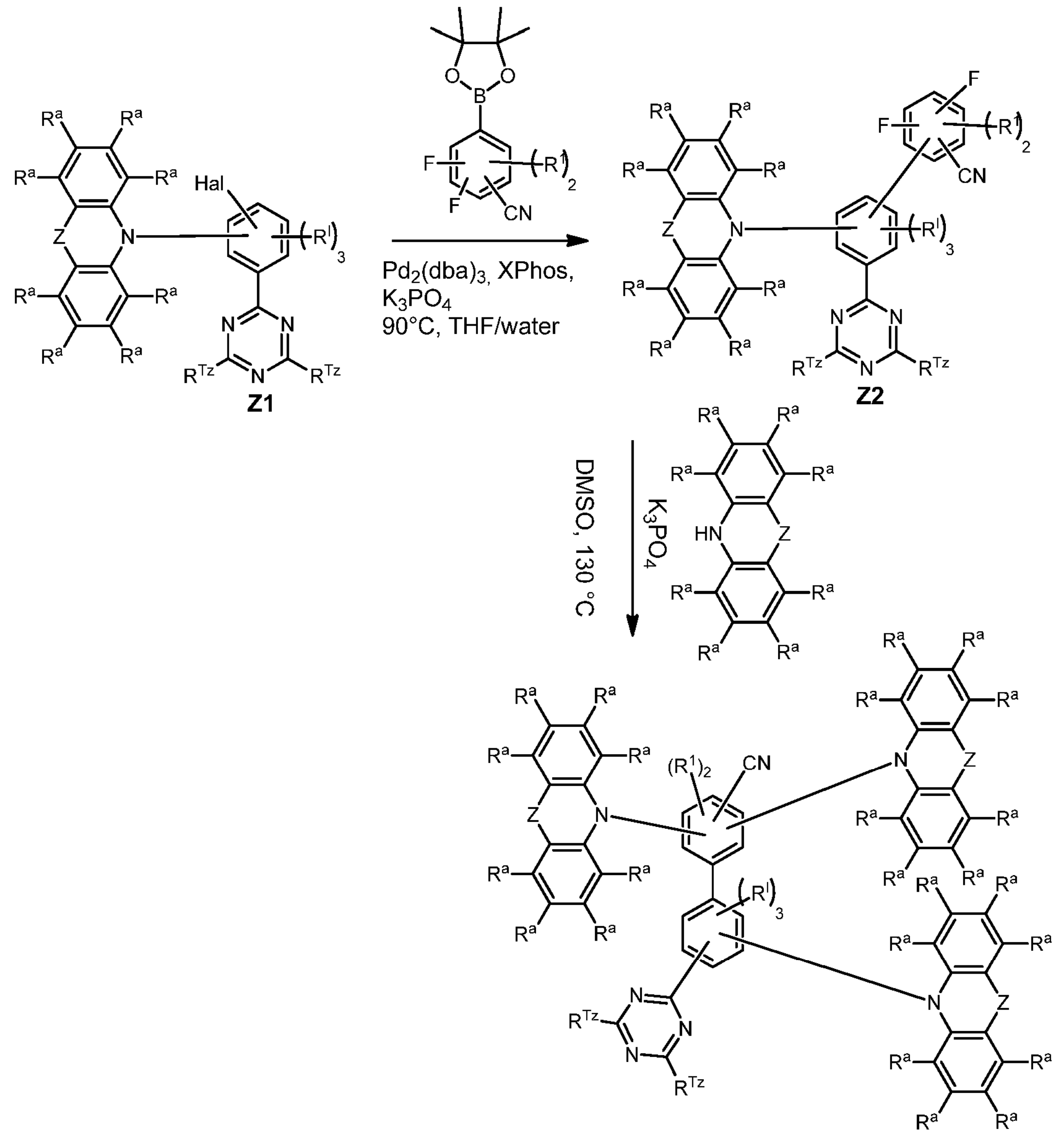


Z1

Z2

For the reaction of a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter or as an absorber, and/or as host material and/or as electron transport material, and/or as hole injection material, and/or as hole blocking material in an optoelectronic device.

The optoelectronic device, also referred to as organic optoelectronic device, may be understood in the broadest sense as any device based on organic materials that is suitable for emitting light in the visible or nearest ultraviolet (UV) range, i.e., in the range of a wavelength of from 380 nm to 800 nm. More preferably, optoelectronic device may be able to emit light in the visible range, i.e., of from 400 nm to 800 nm.

In the context of such use, the optoelectronic device is more particularly selected from the group consisting of:

- organic light-emitting diodes (OLEDs),
- light-emitting electrochemical cells,
- OLED sensors, especially in gas and vapour sensors not hermetically externally shielded,
- organic diodes,
- organic solar cells,

- organic transistors,
- organic field-effect transistors,
- organic lasers, and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention, and
(c) optionally, one or more dyes and/or one or more solvents.

In one embodiment, the light-emitting layer comprises (or essentially consists of) a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises (or essentially consists of) a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally, 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention (E), in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally, 0-94 % by weight, preferably 0.1-65 % by weight, in particular 1-50 % by weight, of at least one further host compound D with a structure differing from the structure of the molecules according to the invention; and
(iv) optionally, 0-94 % by weight, preferably 0-65 % by weight, in particular 0-50 % by weight, of a solvent; and
(v) optionally, 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy

$E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention (E) has a highest occupied molecular orbital HOMO(E) having an energy $E^{HOMO}(E)$ and a lowest unoccupied molecular orbital LUMO(E) having an energy $E^{LUMO}(E)$,

wherein

$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and
$E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the optoelectronic device of the invention, the organic molecule according to the invention E is used as emission material in a light-emitting layer EML.

In one embodiment of the optoelectronic device of the invention the light-emitting layer EML consists of the composition according to the invention described here.

For example, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the optoelectronic device may optionally comprise one or more protective layers protecting the device from damaging exposure to harmful species in the environment including, for example, moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A,

wherein the OLED with an inverted layer structure comprises each layer only optionally, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may, for example, comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

The anode layer A (essentially) may consist of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may, for example, comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl) bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL), a hole transport layer (HTL) is typically located. Herein, any hole transport compound may be used. For example, electronrich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. For example, the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may be used as inorganic dopant. Tetrafluorotetracyanoquinodimethane ($F_4$-TCNQ), copper-pentafluorobenzoate (Cu(I)pFBz) or transition metal complexes may be used as organic dopant.

The EBL may comprise, for example, mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. For example, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl) phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl) phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. For example, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may, for example, comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. For example, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from

the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. For example, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may be an essentially white optoelectronic device. Such a white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, for example, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 $cd/m^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 $cd/m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.45 eV, preferably less than 0.42 eV, more preferably less than 0.40 eV, even more preferably less than 0.38 eV or even less than 0.36 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic device. In this case an organic molecule of the invention is used.

The optoelectronic device, in particular the OLED according to the present invention can be fabricated by any means of vapor deposition and/ or liquid processing. Accordingly, at least one layer is

- prepared by means of a sublimation process,

- prepared by means of an organic vapor phase deposition process,
- prepared by means of a carrier gas sublimation process,
- solution processed or printed.

The methods used to fabricate the optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes may comprise thermal (co)evaporation, chemical vapor deposition and physical vapor deposition. For active matrix OLED display, an AMOLED backplane is used as substrate. The individual layer may be processed from solutions or dispersions employing adequate solvents. A solution deposition process comprises, for example, spin coating, dip coating and jet printing. Liquid processing may optionally be carried out in an inert atmosphere (e.g., in a nitrogen atmosphere) and the solvent may optionally be completely or partially removed by means known in the state of the art.

**Examples**

General synthesis scheme I

Hal = Cl, Br or I

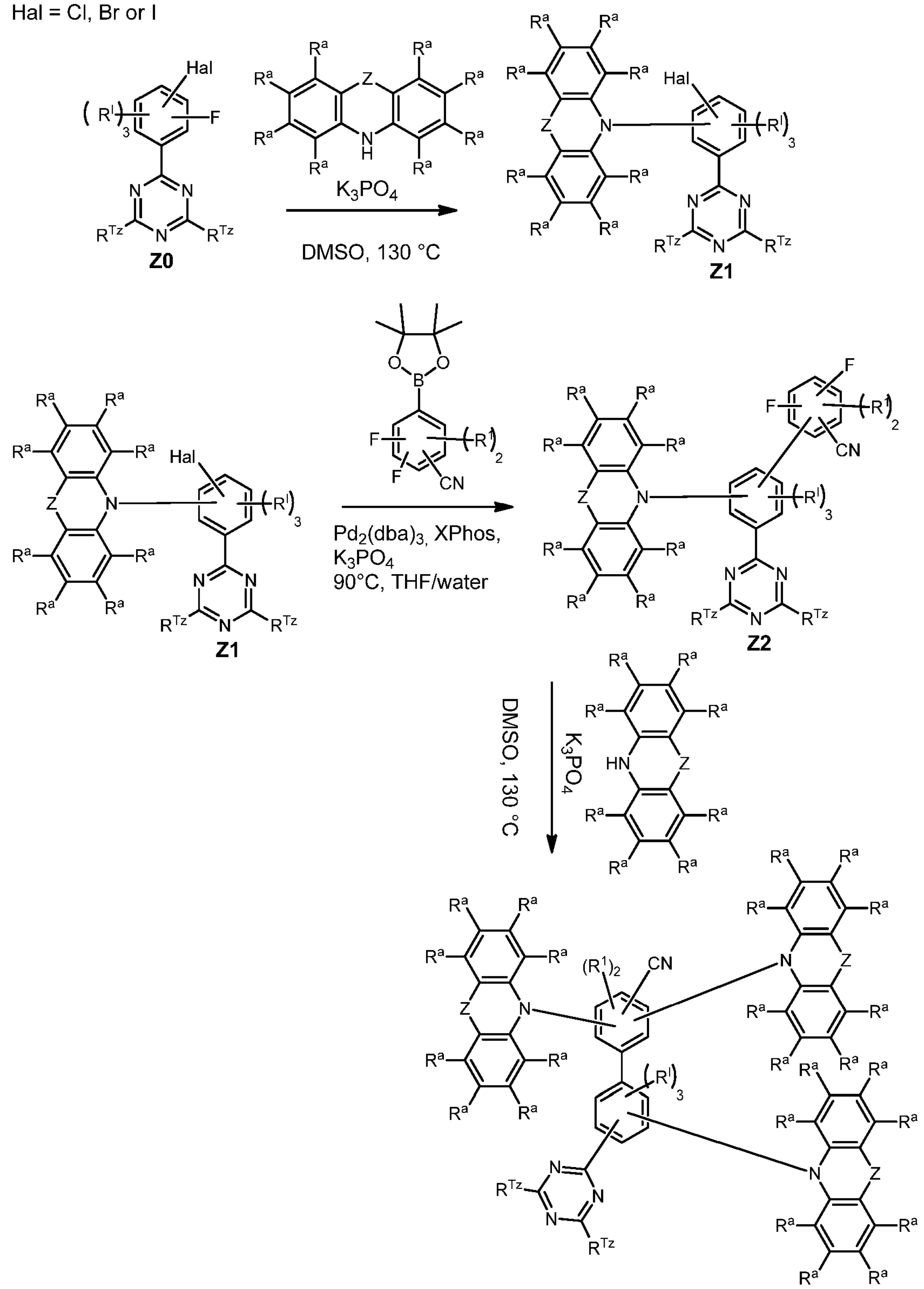


*General procedure for synthesis* ***AAV1:***

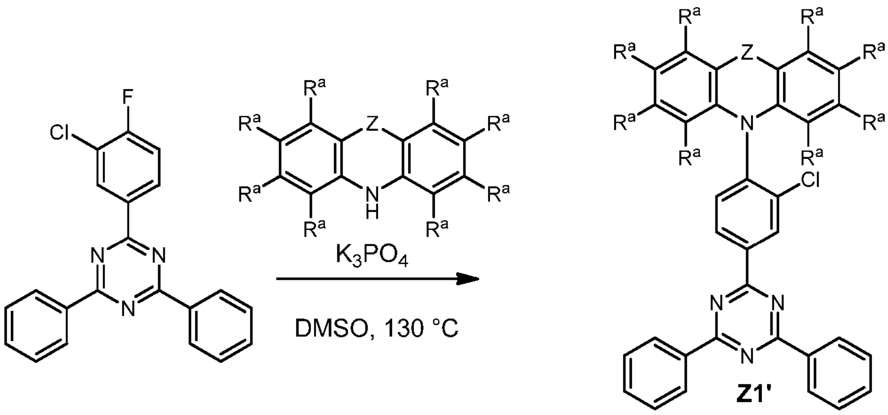


Z1'

2-(3-chloro-4-fluorophenyl)-4,6-diphenyl-1,3,5-triazine (1.00 equivalent, CAS 2243446-04-8), the corresponding donor molecule D-H (1.10 equivalents) and tribasic potassium phosphate (2.00 equivalents) are suspended under nitrogen atmosphere in DMSO and stirred at 130 °C for 12 hours (h). Water was added to the reaction mixture; the product fell out and was filtered off. The crude product Z1' was washed with hot ethanol and filtered off.

*General procedure for synthesis* ***AAV2*** *(not according to the invention):*

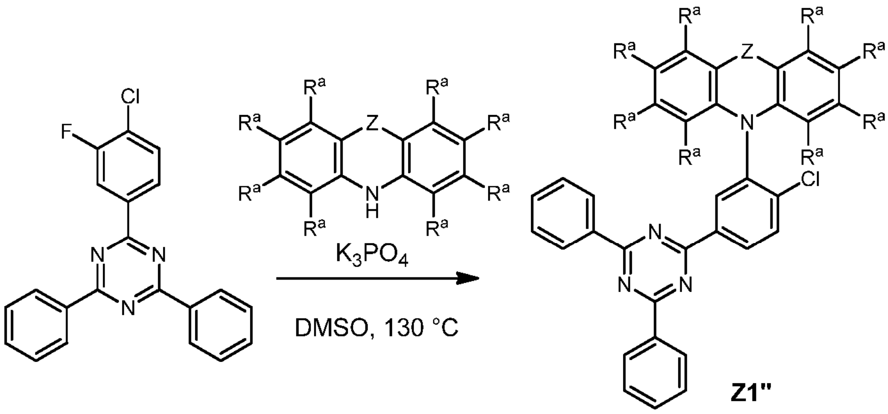


Z1"

2-(4-chloro-3-fluorophenyl)-4,6-diphenyl-1,3,5-triazine (1.00 equivalent), the corresponding donor molecule D-H (1.10 equivalents) and tribasic potassium phosphate (2.00 equivalents) are suspended under nitrogen atmosphere in DMSO and stirred at 130 °C (12 h). Water was added to the reaction mixture; the product fell out and was filtered off. The crude product Z1" was washed with hot ethanol and filtered off.

*General procedure for synthesis* ***AAV3*** *(not according to the invention):*

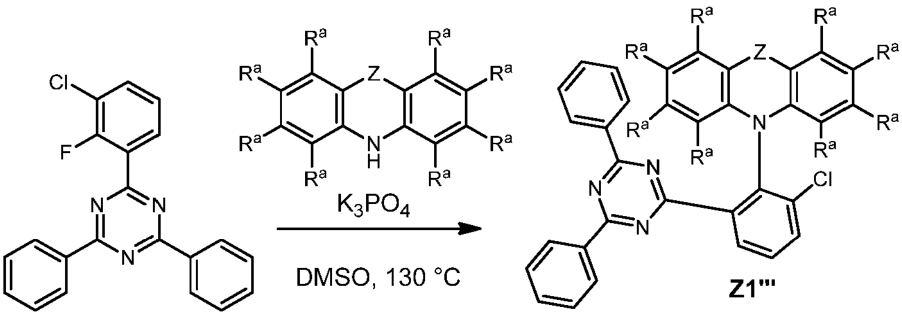


Z1'''

2-(3-chloro-2-fluorophenyl)-4,6-diphenyl-1,3,5-triazine (1.00 equivalent), the corresponding donor molecule D-H (1.10 equivalents) and tribasic potassium phosphate (2.00 equivalents) are suspended under nitrogen atmosphere in DMSO and stirred at 130 °C (12 h). Water was added to the reaction mixture; the product fell out and was filtered off. The crude product Z1‴ was washed with hot ethanol and filtered off.

*General procedure for synthesis **AAV4:***

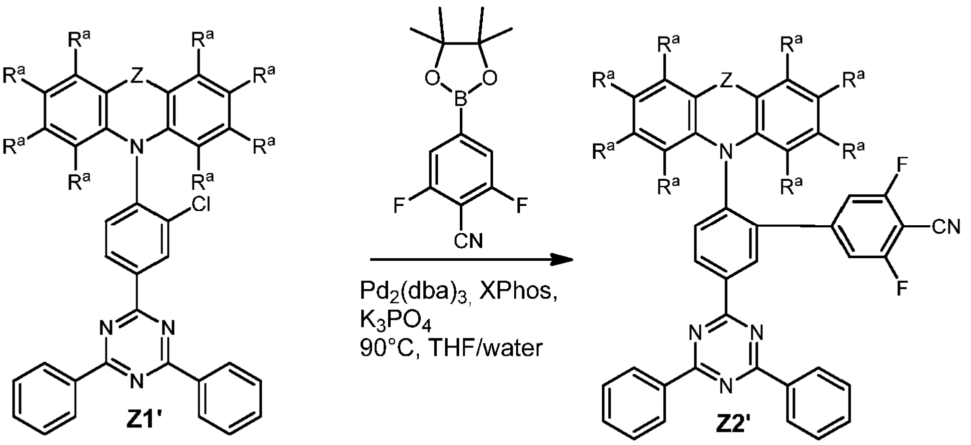


Z1' Z2'

**Z1'** (1.00 equivalent), 2,6-difluoro-4-(boronic acid pinacol ester)-benzonitrile (1.1 equivalents, CAS 1003298-73-4), tris(dibenzylideneacetone)dipalladium(0) $Pd_2(dba)_3$ (0.05 equivalents, CAS 51364-51-3), X-Phos (0.12 equivalents, CAS 564483-18-7) and tribasic potassium phosphate ($K_3PO_4$, 2.0 equivalents) are stirred under nitrogen atmosphere in a tetrahydrofuran (THF)/ water (10:1) mixture at 90 °C for 16 h. After cooling down to room temperature (RT) the reaction mixture is extracted with ethyl acetate/brine. The organic phases are collected, washed with brine and dried over $MgSO_4$. The organic solvent is removed, the crude product was purified via flash chromatography (cyclohexane:ethyl acetate 10:1).

*General procedure for synthesis **AAV5** (not according to the invention):*

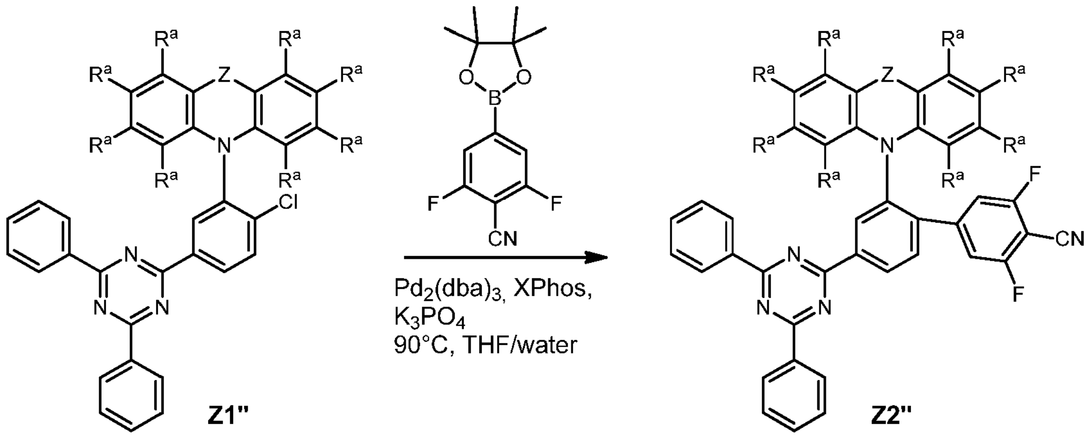


Z1'' Z2''

**Z1''** (1.00 equivalent), 2,6-difluoro-4-(boronic acid pinacol ester)-benzonitrile (1.1 equivalents, CAS 1003298-73-4), tris(dibenzylideneacetone)dipalladium(0) $Pd_2(dba)_3$ (0.05 equivalents, CAS 51364-51-3), X-Phos (0.12 equivalents, CAS 564483-18-7) and tribasic potassium phosphate ($K_3PO_4$, 2.0 equivalents) are stirred under nitrogen atmosphere in a tetrahydrofuran (THF)/ water (10:1) mixture at 90 °C for 16 h. After cooling down to room temperature (RT) the reaction mixture is extracted with ethyl acetate/brine. The organic phases are collected, washed with brine and dried over $MgSO_4$. The organic solvent is removed, the crude product was purified via flash chromatography (cyclohexane:ethyl acetate 10:1).

*General procedure for synthesis **AAV6** (not according to the invention):*

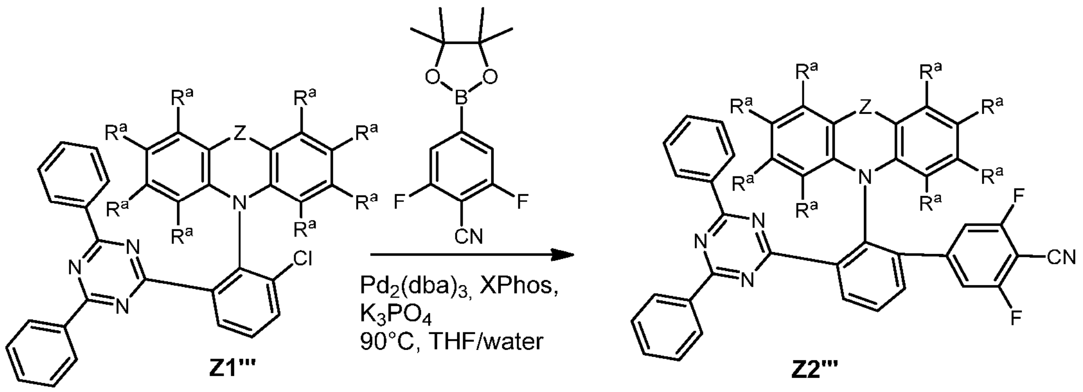


**Z1'''** (1.00 equivalent), 2,6-difluoro-4-(boronic acid pinacol ester)-benzonitrile (1.1 equivalents, CAS 1003298-73-4), tris(dibenzylideneacetone)dipalladium(0) $Pd_2(dba)_3$ (0.05 equivalents, CAS 51364-51-3), X-Phos (0.12 equivalents, CAS 564483-18-7) and tribasic potassium phosphate ($K_3PO_4$, 2.0 equivalents) are stirred under nitrogen atmosphere in a tetrahydrofuran (THF)/ water (10:1) mixture at 90 °C for 16 h. After cooling down to room temperature (RT) the reaction mixture is extracted with ethyl acetate/brine. The organic phases are collected, washed with brine and dried over $MgSO_4$. The organic solvent is removed, the crude product was purified via flash chromatography (cyclohexane:ethyl acetate 10:1).

*General procedure for synthesis **AAV7:***

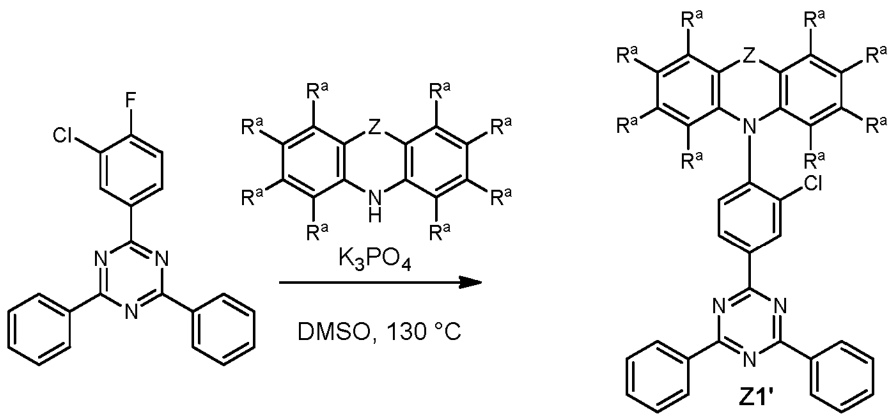


2-(3-chloro-4-fluorophenyl)-4,6-diphenyl-1,3,5-triazine (1.00 equivalent, CAS 2243446-04-8), the corresponding donor molecule D-H (1.10 equivalents) and tribasic potassium phosphate (2.00 equivalents) are suspended under nitrogen atmosphere in DMSO and stirred at 130 °C (12 h). Water was added to the reaction mixture; the product fell out and was filtered off. The crude product Z1' was washed with hot ethanol and filtered off.

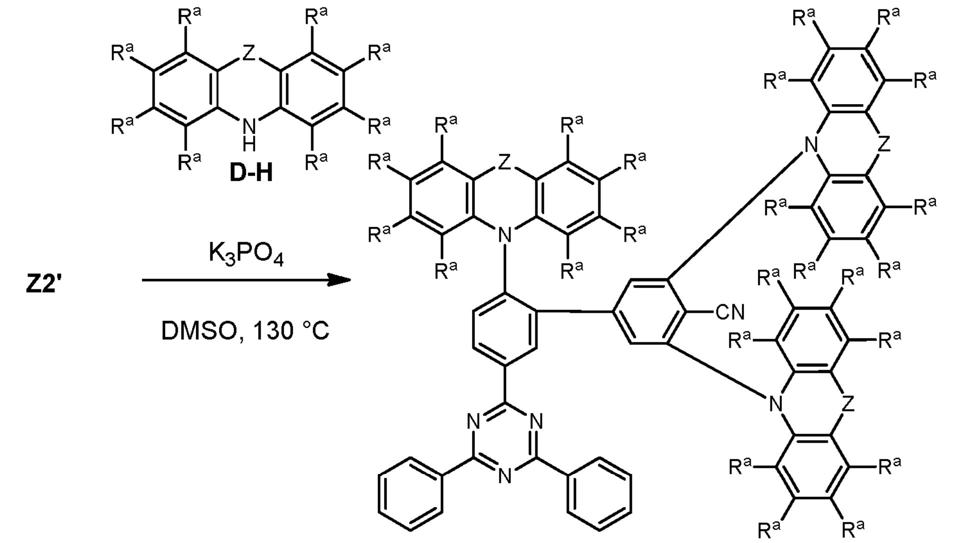


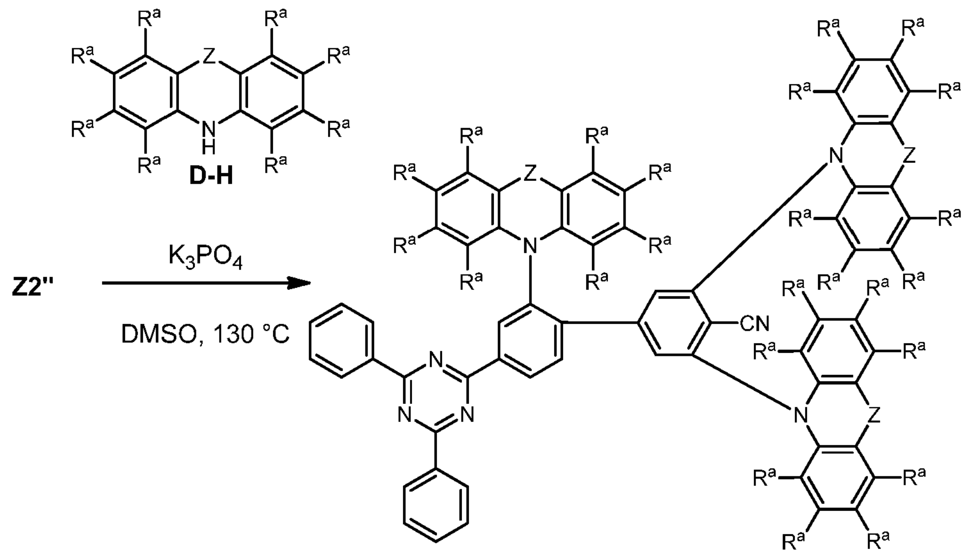


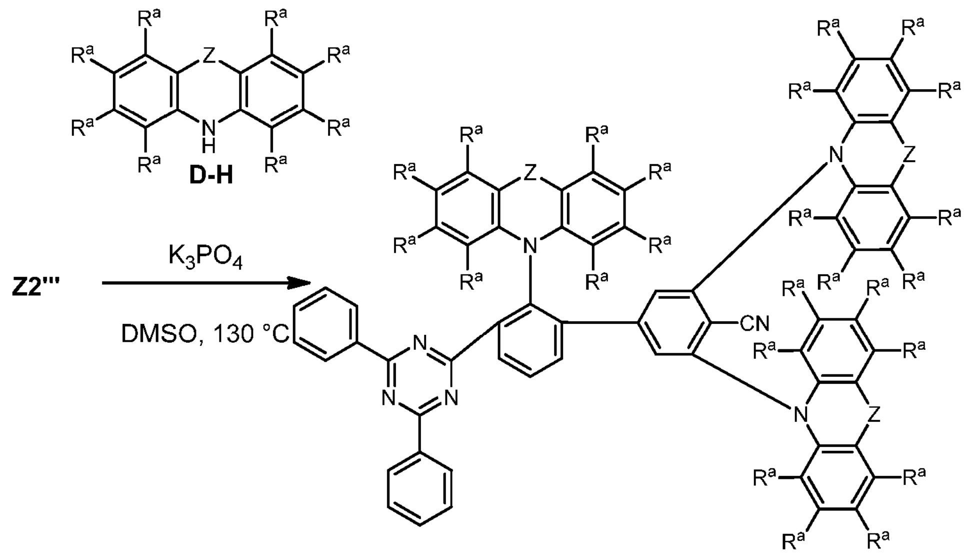


**Z2', Z2"** or **Z2'''** (1.00 equivalent), the corresponding donor molecule D-H (3.00 equivalents) and tribasic potassium phosphate (4.00 equivalents) are suspended under nitrogen atmosphere in DMSO and stirred at 130 °C (12 h). After cooling to room temperature, the reaction mixture is extracted with ethyl acetate/brine. Organic phases are

collected, washed with brine and dried over $MgSO_4$. The solvents are remove under reduced pressure. The crude product is purified by flash chromatography (cyclohexane:ethyl acetate 10:1).

In particular, the donor molecule **D-H** is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

For example, a halogen-substituted carbazole, particularly 3-bromocarbazole, can be used as **D-H.**

In a subsequent reaction, a boronic acid ester functional group or boronic acid functional group may be, for example, introduced at the position of the one or more halogen substituents, which was introduced via D-H, to yield the corresponding carbazol-3-ylboronic acid ester or carbazol-3-ylboronic acid, e.g., via the reaction with bis(pinacolato) diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$ [$R^a$-$B(OH)_2$] or a corresponding boronic acid ester.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.

Program: 1) 3 s at 400 U/min; 20 s at 1000 U/min at 1000 Upm/s. 3) 10 s at 4000 U/min at 1000 Upm/s. After coating, the films are tried at 70 °C for 1 min.

Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.

Excitation sources:

NanoLED 370 (wavelength: 371 nm, puls duration: 1,1 ns)
NanoLED 290 (wavelength: 294 nm, puls duration: <1 ns)

SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement* C9920-03G system (*Hamamatsu Photonics*) is used. Quantum yields and CIE coordinates are determined using the software U6039-05 version 3.6.0.

Emission maxima are given in nm, quantum yields Φ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement

Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**HPLC-MS:**

HPLC-MS analysis is performed on an HPLC by Agilent (1100 series) with MS-detector (Thermo LTQ XL).

A typical HPLC method is as follows: a reverse phase column 4,6mm x 150mm, particle size 3,5 μm from Agilent *(ZORBAX Eclipse Plus 95Å C18, 4.6 x 150 mm, 3.5 μm HPLC column)* is used in the HPLC. The HPLC-MS measurements are performed at room temperature (rt) following gradients

| Flow rate [ml/min] | time [min] | A[%] | B[%] | C[%] |
|---|---|---|---|---|
| 2.5 | 0 | 40 | 50 | 10 |
| 2.5 | 5 | 40 | 50 | 10 |
| 2.5 | 25 | 10 | 20 | 70 |
| 2.5 | 35 | 10 | 20 | 70 |
| 2.5 | 35.01 | 40 | 50 | 10 |
| 2.5 | 40.01 | 40 | 50 | 10 |
| 2.5 | 41.01 | 40 | 50 | 10 |

using the following solvent mixtures:

| | | |
|---|---|---|
| Solvent A: | $H_2O$ (90%) | MeCN (10%) |
| Solvent B: | $H_2O$ (10%) | MeCN (90%) |
| Solvent C: | THF (50%) | MeCN (50%) |

An injection volume of 5 μL from a solution with a concentration of 0.5 mg/mL of the analyte is taken for the measurements.

Ionization of the probe is performed using an APCI (atmospheric pressure chemical ionization) source either in

positive (APCI +) or negative (APCI -) ionization mode.

Production and characterization of optoelectronic devices

Optoelectronic devices, such as OLED devices, comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.

The (not fully optimized) OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given.

**Example 1**

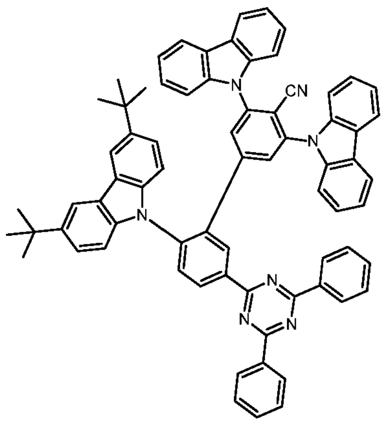

Example 1 was synthesized according to ***AAV1*** (yield = 80%), ***AAV4*** (yield = 71%) and ***AAV7*** (yield = 23 %).

HPLC-MS: 26.02 min (1017.35 m/z, 100 %)).

Figure 1 depicts the emission spectrum of example 1 (10 % by weight in PMMA). The emission maximum ($\lambda_{max}$) is at 462 nm. The photoluminescence quantum yield (PLQY) is 80 %, the full width at half maximum (FWHM) is 0.38 eV. The corresponding CIEy coordinate is 0.18.

Additional Examples of Organic Molecules of the Invention

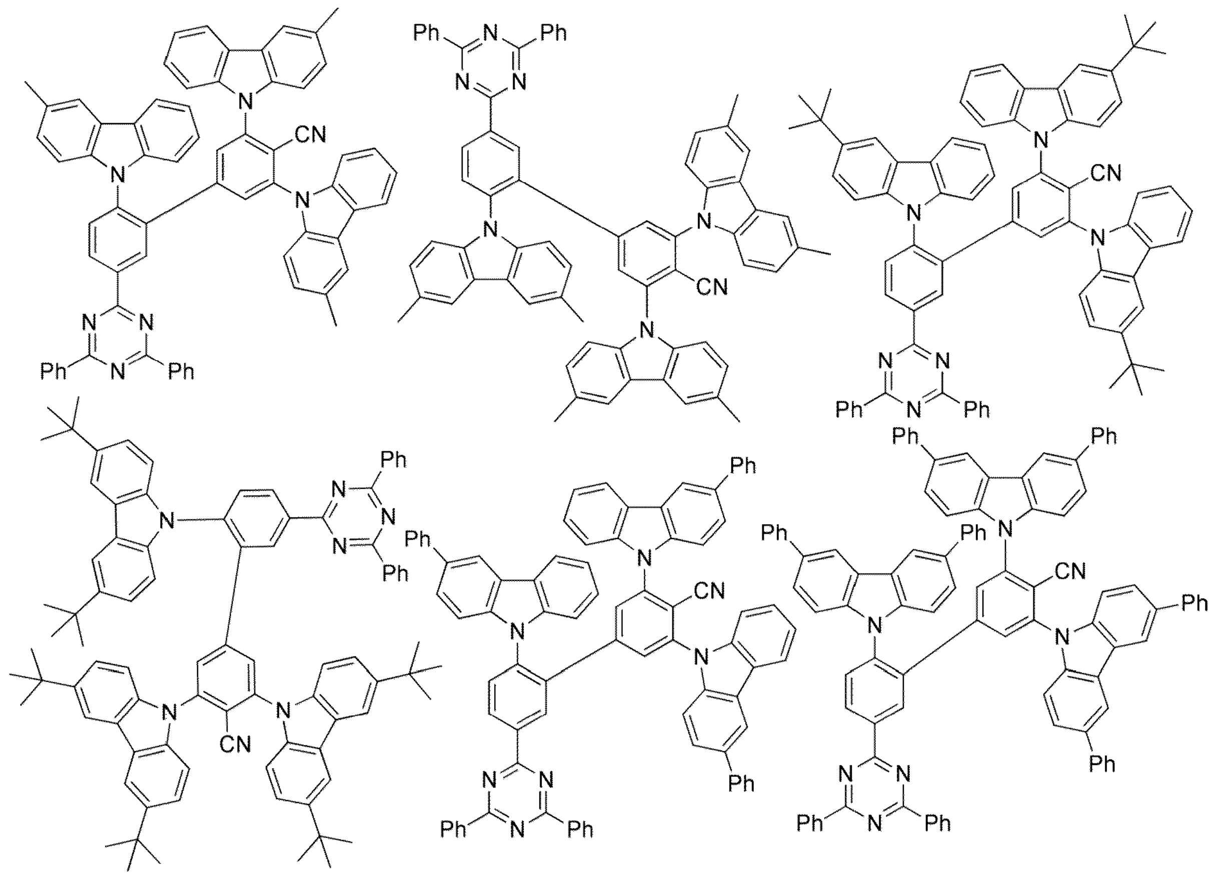

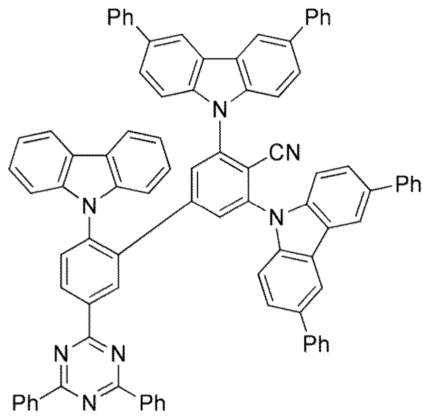

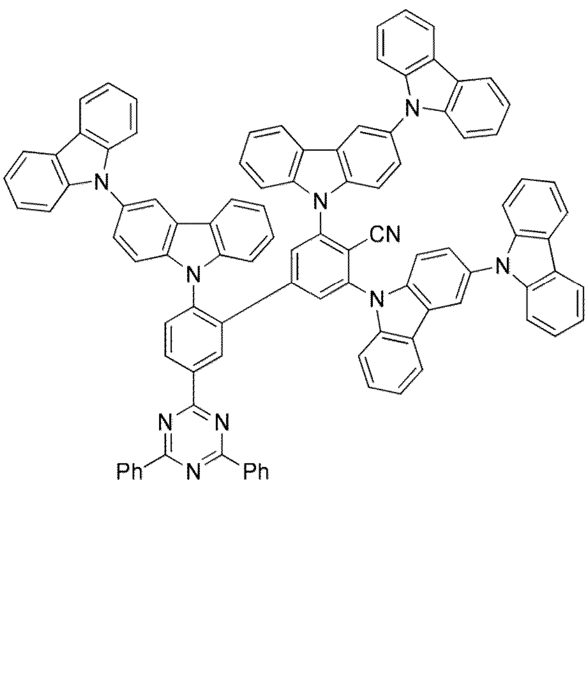

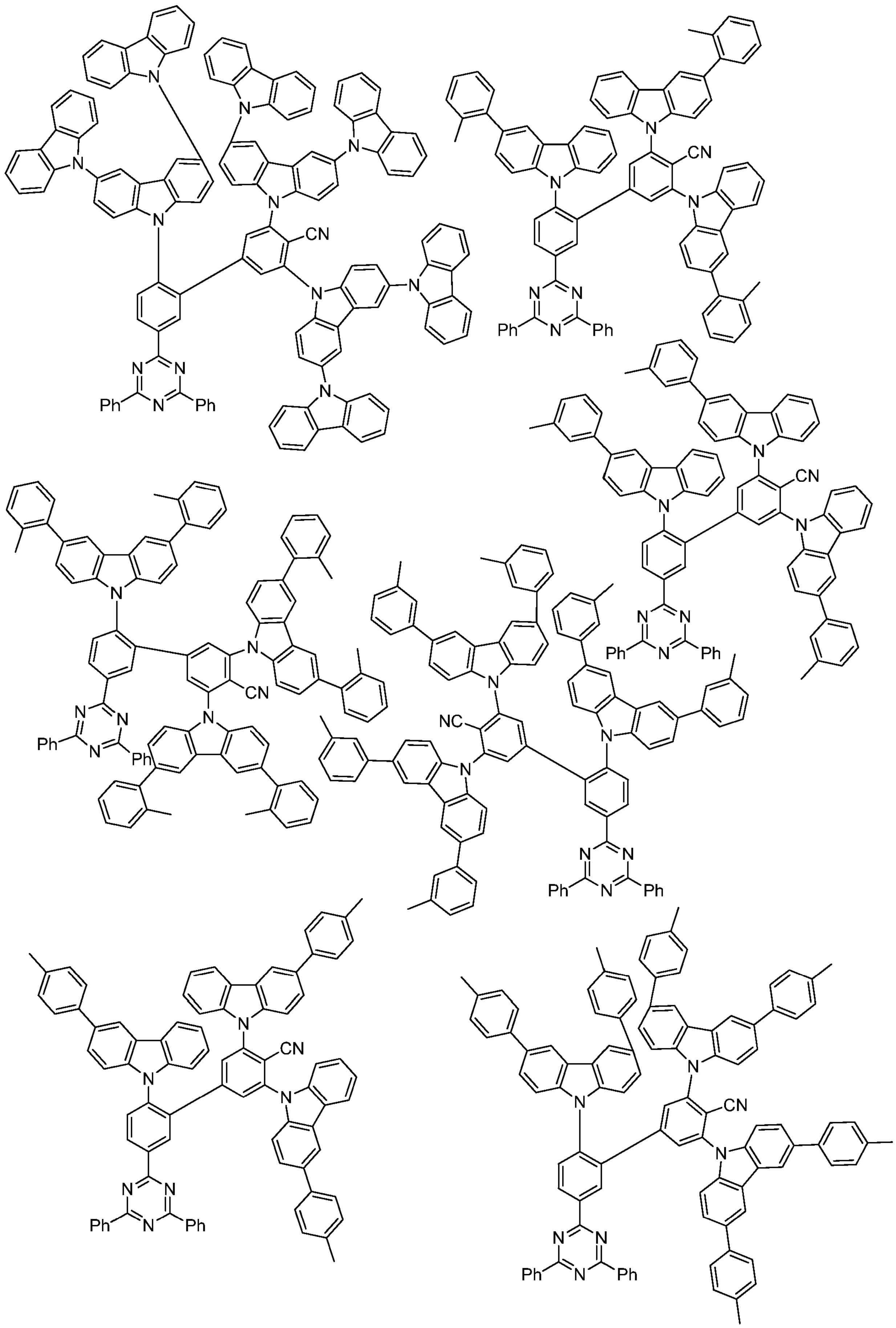

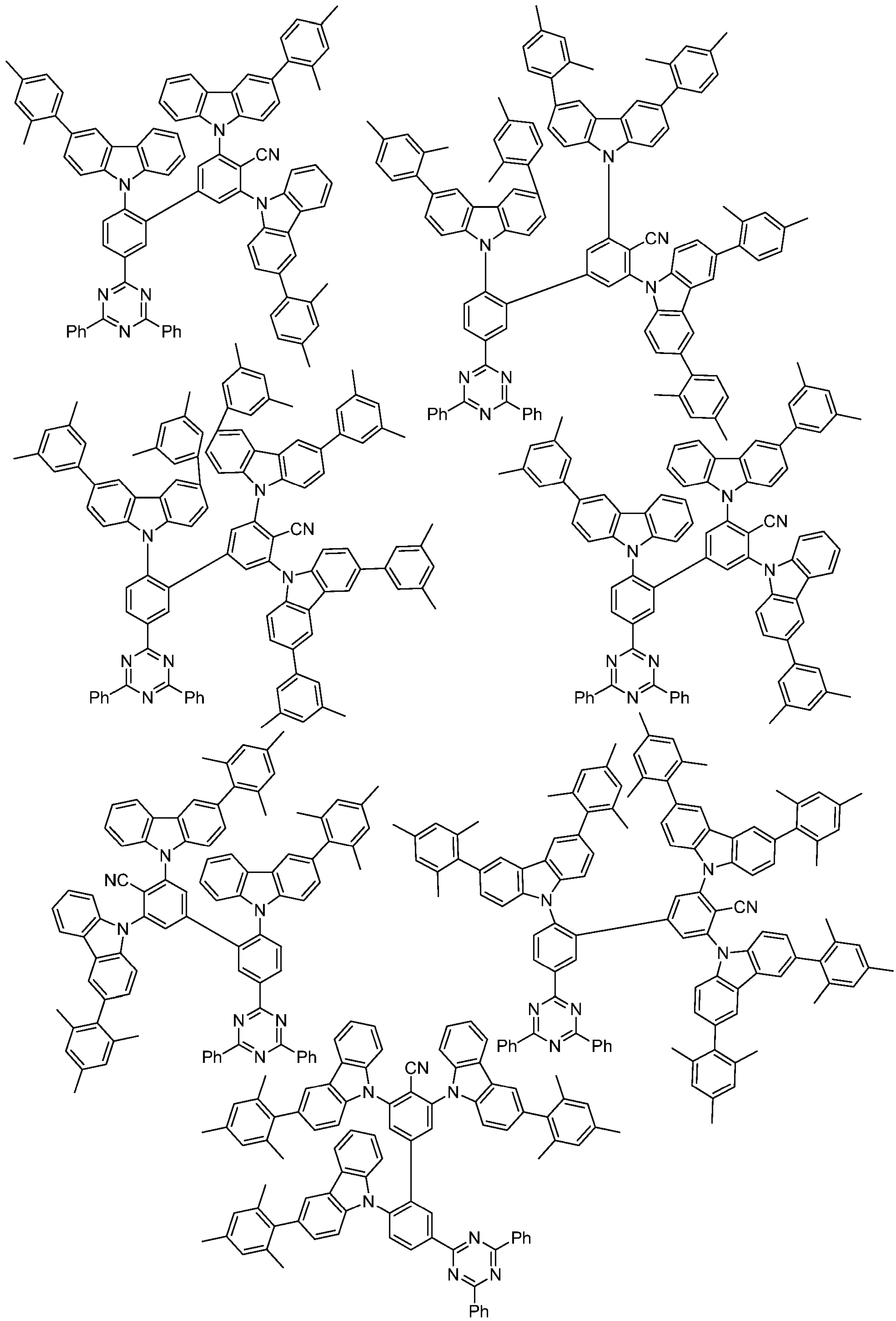

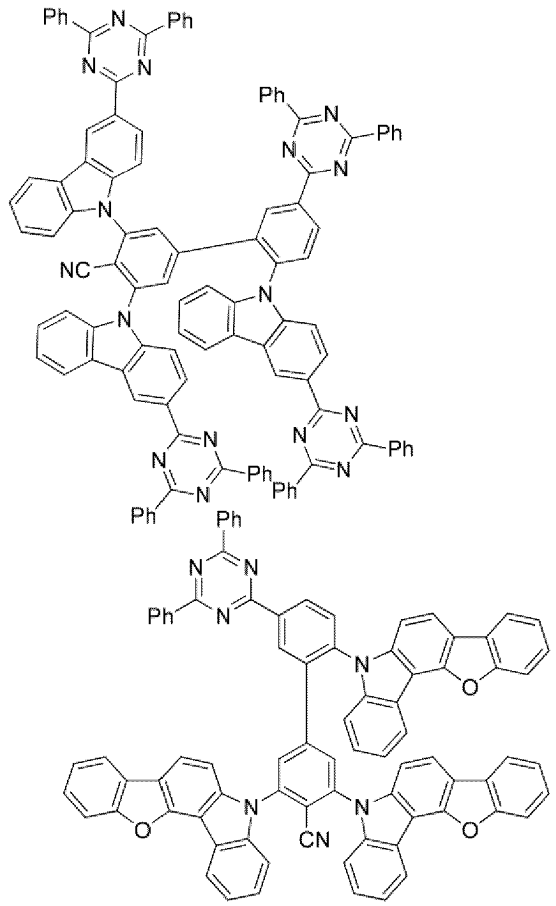

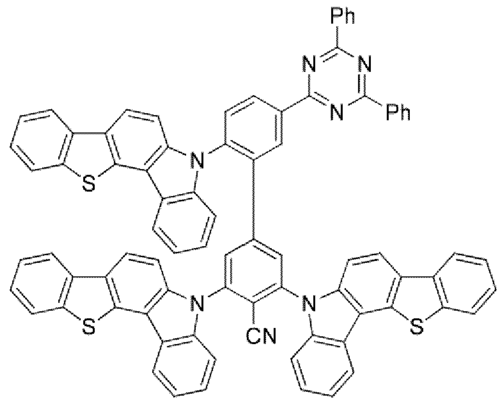

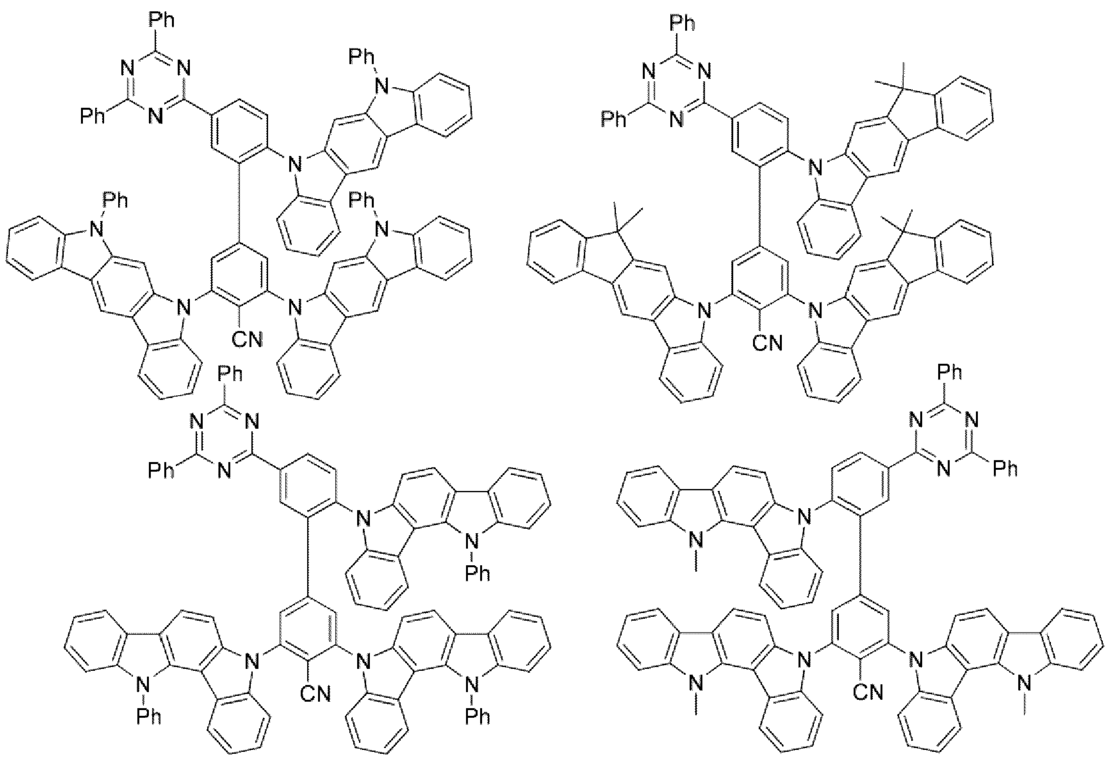

Figures

Figure 1 Emission spectrum of example 1 (10% by weight) in PMMA.

**Claims**

**1.** Organic molecule, comprising

- one first chemical moiety comprising a structure of formula Iaa,

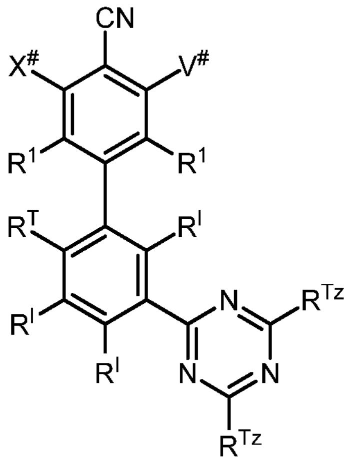

Formula Iaa

and
- three second chemical moieties, each independently comprising a structure of formula II,

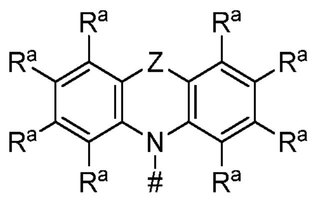

Formula II

wherein the first chemical moiety is linked to each of the three second chemical moieties via a single bond;
wherein
$X^{\#}$ is the binding site of a single bond linking the first chemical moiety to one of the three second chemical moieties,
$V^{\#}$ is the binding site of a single bond linking the first chemical moiety to one of the three second chemical moieties,
$R^T$ is the binding site of a single bond linking the first chemical moiety to one of the three second chemical moieties;
# represents the binding site of a single bond linking the second chemical moieties to the first chemical moiety;
Z is a direct bond;
$R^1$ is at each occurrence independently selected from the group consisting of:

hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and

$C_6$-$C_{18}$-aryl,
which is optionally substituted with one or more substituents $R^6$;
$R^I$ is at each occurrence independently selected from the group consisting of:

hydrogen, deuterium,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$;
$R^{Tz}$ is at each occurrence independently selected from the group consisting of hydrogen, deuterium,
$C_1$-$C_5$-alkyl,
wherein one or more hydrogen atoms are optionally substituted by deuterium;
$C_6$-$C_{18}$-aryl,
which is optionally substituted with one or more substituents $R^{6'}$; and
$R^{6'}$ is at each occurrence independently selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$N(C_6\text{-}C_{18}\text{-aryl})_2$;
$N(C_3\text{-}C_{17}\text{-heteroaryl})_2$, and
$N(C_3$-$C_{17}$-heteroaryl)($C_6$-$C_{18}$-aryl);
$R^a$ is at each occurrence independently selected from the group consisting of:

hydrogen, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
$R^5$ is at each occurrence independently selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and

wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$;
$R^6$ is at each occurrence independently selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$, and
$N(C_3$-$C_{17}$-heteroaryl$)(C_6$-$C_{18}$-aryl);
wherein the substituents $R^a$ or $R^5$ independently from each other optionally form a mono- or polycyclic, aliphatic, aromatic and/or benzo-fused ring system with one or more substituents $R^a$ or $R^5$;
wherein the terms alkyl, alkenyl, alkynyl, alkoxy and thioalkoxy can refer to linear, branched, and cyclic substituents.

2. Organic molecule according to claim 1, wherein $R^I$ and $R^1$ is independently from each other selected from the group of consisting of hydrogen (H), methyl, mesityl, tolyl, and phenyl.

3. Organic molecule according to one or more of claims 1 or 2, wherein the three second chemical moieties each at each occurrence, independently from another, comprise a structure of formula IIb:

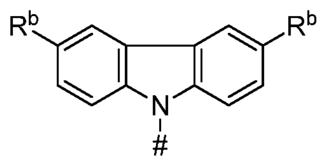

Formula IIb

wherein

$R^b$ is at each occurrence independently selected from the group consisting of: H, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$, and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$, and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$, and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$, and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**4.** Organic molecule according to one or more of claims 1 to 3, wherein the three second chemical moieties each at each occurrence, independently from another, comprise a structure of formula IIc:

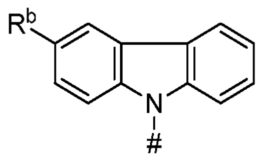

Formula IIc

wherein

$R^b$ is at each occurrence independently selected from the group consisting of H, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$, and
wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$,

$C_1$-$C_{40}$-alkoxy,

which is optionally substituted with one or more substituents $R^5$ and

wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**5.** Organic molecule according to claim 3 or 4, wherein $R^b$ is at each occurrence independently selected from the group consisting of

- H,
- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidinyl, which is optionally substituted with one or more substituents independently from each other selected from the group consisting of Me, $^i$Pr, $^t$Bu, CN, $CF_3$ and Ph;
- carbazolyl, which is optionally substituted with one or more substituents independently from each other selected from the group consisting of Me, $^i$Pr, $^t$Bu, CN, $CF_3$ and Ph;
- triazinyl, which is optionally substituted with one or more substituents independently from each other selected from the group consisting of Me, $^i$Pr, $^t$Bu, CN, $CF_3$, and Ph;

and
- $N(Ph)_2$.

**6.** Use of an organic molecule according to one or more of claims 1 to 5 as a luminescent emitter and/or as a host material and/or as an electron transport material and/or as a hole injection material and/or as a hole blocking material in an optoelectronic device.

**7.** Use according to claim 6, wherein the optoelectronic device is selected from the group consisting of:

• organic light-emitting diodes (OLEDs),
• light-emitting electrochemical cells,
• OLED-sensors,
• organic diodes,
• organic solar cells,
• organic transistors,
• organic field-effect transistors,

• organic lasers and
• down-conversion elements.

**8.** Composition, comprising:

(a) an organic molecule according to one or more of claims 1 to 5, in particular in the form of an emitter and/or a host, and
(b) an emitter and/or host material, which differs from the organic molecule of one or more of claims 1 to 5, and
(c) optionally, a dye and/or a solvent.

**9.** Optoelectronic device, comprising an organic molecule according to one or more of claims 1 to 5 or a composition according to claim 8, in particular in the form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**10.** Optoelectronic device according to claim 9, comprising:

- a substrate,
- an anode, and
- a cathode, wherein the anode or the cathode are disposed on the substrate, and
- a light-emitting layer, which is arranged between the anode and the cathode and which comprises the organic molecule or the composition.

**11.** Method for producing an optoelectronic device, wherein an organic molecule according to one or more of claims 1 to 5 or a composition according to claim 8 is used.

**12.** Method according to claim 11, comprising processing of the organic molecule by a vacuum evaporation method or from a solution.

**Patentansprüche**

**1.** Organisches Molekül, umfassend

- eine erste chemische funktionelle Gruppe, umfassend eine Struktur der Formel Iaa

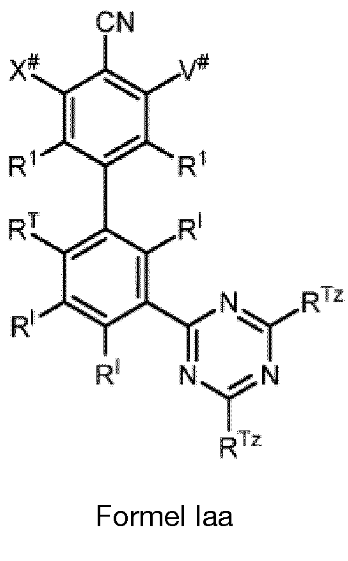

Formel Iaa

und
- drei zweite chemische funktionelle Gruppen, die jeweils unabhängig eine Struktur der Formel II umfassen

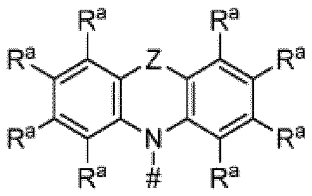

Formel II

wobei die erste chemische funktionelle Gruppe mit jeder der drei zweiten chemischen funktionellen Gruppen über eine einzelne Bindung verknüpft ist;

wobei $X^{\#}$ die Bindungsstelle einer einzelnen Bindung ist, die die erste chemische funktionelle Gruppe mit einer der drei zweiten chemischen funktionellen Gruppen verknüpft, $V^{\#}$ die Bindungsstelle einer einzelnen Bindung ist, die die erste chemische funktionelle Gruppe mit einer der drei zweiten chemischen funktionellen Gruppen verknüpft, $R^T$ die Bindungsstelle einer einzelnen Bindung ist, die die erste chemische funktionelle Gruppe mit einer der drei zweiten chemischen funktionellen Gruppen verknüpft;

# die Bindungsstelle einer einzelnen Bindung darstellt, die die zweiten chemischen funktionellen Gruppen mit der ersten chemischen funktionellen Gruppe verknüpft; Z eine direkte Bindung ist;

$R^1$ bei jedem Auftreten unabhängig ausgewählt ist aus der Gruppe bestehend aus: Wasserstoff, Deuterium, $C_1$-$C_5$-Alkyl, wobei ein oder mehrere Wasserstoffatome gegebenenfalls durch Deuterium substituiert sind; $C_2$-$C_8$-Alkenyl, wobei ein oder mehrere Wasserstoffatome gegebenenfalls durch Deuterium substituiert sind; $C_2$-$C_8$-Alkinyl, wobei ein oder mehrere Wasserstoffatome gegebenenfalls durch Deuterium substituiert sind; und $C_6$-$C_{18}$-Aryl, das gegebenenfalls mit einem oder mehreren Substituenten $R^6$ substituiert ist; $R^I$ bei jedem Auftreten unabhängig ausgewählt ist aus der Gruppe bestehend aus: Wasserstoff, Deuterium, $C_1$-$C_5$-Alkyl, wobei ein oder mehrere Wasserstoffatome gegebenenfalls durch Deuterium substituiert sind; $C_2$-$C_8$-Alkenyl, wobei ein oder mehrere Wasserstoffatome gegebenenfalls durch Deuterium substituiert sind; $C_2$-$C_8$-Alkinyl, wobei ein oder mehrere Wasserstoffatome gegebenenfalls durch Deuterium substituiert sind;

und $C_6$-$C_{18}$-Aryl, das gegebenenfalls mit einem oder mehreren Substituenten $R^6$ substituiert ist; $R^{Tz}$ bei jedem Auftreten unabhängig ausgewählt ist aus der Gruppe bestehend aus Wasserstoff, Deuterium, $C_1$-$C_5$-Alkyl, wobei ein oder mehrere Wasserstoffatome gegebenenfalls durch Deuterium substituiert sind; $C_6$-$C_{18}$-Aryl, das gegebenenfalls mit einem oder mehreren Substituenten $R^{6'}$ substituiert ist; und $R^{6'}$ bei jedem Auftreten unabhängig ausgewählt ist aus der Gruppe bestehend aus:

Wasserstoff, Deuterium, OPh, $CF_3$, CN, F, $C_1$-$C_5$-Alkyl,
wobei ein oder mehrere Wasserstoffatome gegebenenfalls unabhängig voneinander durch Deuterium, CN, $CF_3$ oder F substituiert sind;
$C_1$-$C_5$-Alkoxy, wobei ein oder mehrere Wasserstoffatome gegebenenfalls unabhängig voneinander durch Deuterium, CN, $CF_3$ oder F substituiert sind;
$C_1$-$C_5$-Thioalkoxy, wobei ein oder mehrere Wasserstoffatome gegebenenfalls unabhängig voneinander durch Deuterium, CN, $CF_3$ oder F substituiert sind;
$C_2$-$C_5$-Alkenyl, wobei ein oder mehrere Wasserstoffatome gegebenenfalls unabhängig voneinander durch Deuterium, CN, $CF_3$ oder F substituiert sind;
$C_2$-$C_5$-Alkinyl, wobei ein oder mehrere Wasserstoffatome gegebenenfalls unabhängig voneinander durch Deuterium, CN, $CF_3$ oder F substituiert sind;
$C_6$-$C_{18}$-Aryl, das gegebenenfalls mit einem oder mehreren $C_1$-$C_5$-Alkylsubstituenten substituiert ist; $N(C_6$-$C_{18}$-Aryl$)_2$; $N(C_3$-$C_{17}$-Heteroaryl$)_2$ und
$N(C_3$-$C_{17}$-Heteroaryl$)(C_6$-$C_{18}$-aryl); $R^a$ bei jedem Auftreten unabhängig ausgewählt ist aus der Gruppe bestehend aus:

Wasserstoff, Deuterium, $N(R^5)_2$, $OR^5$, $Si(R^5)_3$, $B(OR^5)_2$, $OSO_2R^5$, $CF_3$, CN, F, Br, I, $C_1$-$C_{40}$-Alkyl, das gegebenenfalls mit einem oder mehreren Substituenten $R^5$ substituiert ist, und wobei eine oder mehrere nicht benachbarte $CH_2$-Gruppen gegebenenfalls durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ substituiert sind;
$C_1$-$C_{40}$-Alkoxy, das gegebenenfalls mit einem oder mehreren Substituenten $R^5$ substituiert ist, und wobei eine oder mehrere nicht benachbarte $CH_2$-Gruppen gegebenenfalls durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ substituiert sind;
$C_1$-$C_{40}$-Thioalkoxy, das gegebenenfalls mit einem oder mehreren Substituenten $R^5$ substituiert ist, und

wobei eine oder mehrere nicht benachbarte $CH_2$-Gruppen gegebenenfalls durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;

$C_2$-$C_{40}$-Alkenyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;

$C_2$-$C_{40}$-Alkinyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;

$C_6$-$C_{60}$-Aryl, das gegebenenfalls mit einem oder mehreren Substituenten $R^5$ substituiert ist; und $C_3$-$C_{57}$-Heteroaryl,

das gegebenenfalls mit einem oder mehreren Substituenten $R^5$ substituiert ist;

$R^5$ bei jedem Auftreten unabhängig ausgewählt ist aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I, $C_1$-$C_{40}$-Alkyl, das gegebenenfalls mit einem oder mehreren Substituenten $R^6$ substituiert ist, und

wobei eine oder mehrere nicht benachbarte $CH_2$-Gruppen gegebenenfalls durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;

$C_1$-$C_{40}$-Alkoxy, das gegebenenfalls mit einem oder mehreren Substituenten $R^6$ substituiert ist, und wobei eine oder mehrere nicht benachbarte $CH_2$-Gruppen gegebenenfalls durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;

$C_1$-$C_{40}$-Thioalkoxy, das gegebenenfalls mit einem oder mehreren Substituenten $R^6$ substituiert ist, und wobei eine oder mehrere nicht benachbarte $CH_2$-Gruppen gegebenenfalls durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;

$C_2$-$C_{40}$-Alkenyl, das gegebenenfalls mit einem oder mehreren Substituenten $R^6$ substituiert ist, und wobei eine oder mehrere nicht benachbarte $CH_2$-Gruppen gegebenenfalls durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;

$C_2$-$C_{40}$-Alkinyl, das gegebenenfalls mit einem oder mehreren Substituenten $R^6$ substituiert ist, und wobei eine oder mehrere nicht benachbarte $CH_2$-Gruppen gegebenenfalls durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;

$C_6$-$C_{60}$-Aryl, das gegebenenfalls mit einem oder mehreren Substituenten $R^6$ substituiert ist; und $C_3$-$C_{57}$-Heteroaryl, das gegebenenfalls mit einem oder mehreren Substituenten $R^6$ substituiert ist;

$R^6$ bei jedem Auftreten unabhängig ausgewählt ist aus der Gruppe bestehend aus:

Wasserstoff, Deuterium, OPh, $CF_3$, CN, F, $C_1$-$C_5$-Alkyl, wobei ein oder mehrere Wasserstoffatome gegebenenfalls unabhängig voneinander durch Deuterium, CN, $CF_3$ oder F substituiert sind; $C_1$-$C_5$-Alkoxy, wobei ein oder mehrere Wasserstoffatome gegebenenfalls unabhängig voneinander durch Deuterium, CN, $CF_3$ oder F substituiert sind; $C_1$-$C_5$-Thioalkoxy,

wobei ein oder mehrere Wasserstoffatome gegebenenfalls unabhängig voneinander durch Deuterium, CN, $CF_3$ oder F substituiert sind;

$C_2$-$C_5$-Alkenyl, wobei ein oder mehrere Wasserstoffatome gegebenenfalls unabhängig voneinander durch Deuterium, CN, $CF_3$ oder F substituiert sind;

$C_2$-$C_5$-Alkinyl, wobei ein oder mehrere Wasserstoffatome gegebenenfalls unabhängig voneinander durch Deuterium, CN, $CF_3$ oder F substituiert sind;

$C_6$-$C_{18}$-Aryl, das gegebenenfalls mit einem oder mehreren $C_1$-$C_5$-Alkylsubstituenten substituiert ist; $C_3$-$C_{17}$-Heteroaryl,

das gegebenenfalls mit einem oder mehreren $C_1$-$C_5$-Alkylsubstituenten substituiert ist; $N(C_6$-$C_{18}$-$Aryl)_2$; $N(C_3$-$C_{17}$-$Heteroaryl)_2$ und

$N(C_3$-$C_{17}$-Heteroaryl$)(C_6$-$C_{18}$-aryl); wobei die Substituenten $R^a$ oder $R^5$ unabhängig voneinander gegebenenfalls ein mono- oder polycyclisches, aliphatisches, aromatisches, heteroaromatisches und/oder benzokondensiertes Ringsystem mit einem oder mehreren Substituenten $R^a$ oder $R^5$ bilden;

wobei sich die Begriffe Alkyl, Alkenyl, Alkinyl, Alkoxy und Thioalkoxy auf lineare, verzweigte und cyclische Substituenten beziehen können.

**2.** Organisches Molekül nach Anspruch 1, wobei $R^1$ und $R^1$ unabhängig voneinander ausgewählt sind aus der Gruppe bestehend aus Wasserstoff (H), Methyl, Mesityl, Tolyl und Phenyl.

**3.** Organisches Molekül nach Anspruch 1 oder 2, wobei die drei zweiten chemischen funktionellen Gruppen jeweils bei jedem Auftreten unabhängig voneinander eine Struktur der Formel IIb umfassen:

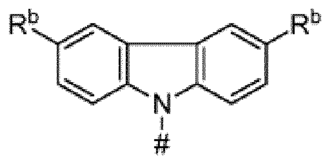

Formel IIb

wobei

$R^b$ bei jedem Auftreten unabhängig ausgewählt ist aus der Gruppe bestehend aus: H, Deuterium, $N(R^5)_2$, $OR^5$, $Si(R^5)_3$, $B(OR^5)_2$, $OSO_2R^5$, $CF_3$, CN, F, Br, I, $C_1$-$C_{40}$-Alkyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkinyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_6$-$C_{60}$-Aryl, das gegebenenfalls mit einem oder mehreren Substituenten $R^5$ substituiert ist; und $C_3$-$C_{57}$-Heteroaryl,
das gegebenenfalls mit einem oder mehreren Substituenten $R^5$ substituiert ist; und wobei davon abgesehen die Definitionen in Anspruch 1 gelten.

**4.** Organisches Molekül nach einem der Ansprüche 1 bis 3, wobei die drei zweiten chemischen funktionellen Gruppen jeweils bei jedem Auftreten unabhängig voneinander eine Struktur der Formel IIc umfassen:

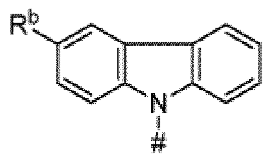

Formel IIc

wobei

$R^b$ bei jedem Auftreten unabhängig ausgewählt ist aus der Gruppe bestehend aus H, Deuterium, $N(R^5)_2$, $OR^5$, $Si(R^5)_3$, $B(OR^5)_2$, $OSO_2R^5$, $CF_3$, CN, F, Br, I, $C_1$-$C_{40}$-Alkyl, das gegebenenfalls mit einem oder mehreren Substituenten $R^5$ substituiert ist, und wobei eine oder mehrere nicht benachbarte $CH_2$-Gruppen gegebenenfalls

durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy, das gegebenenfalls mit einem oder mehreren Substituenten $R^5$ substituiert ist, und wobei eine oder mehrere nicht benachbarte $CH_2$-Gruppen gegebenenfalls substituiert sind durch
$R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_1$-$C_{40}$-Thioalkoxy, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkinyl, das gegebenenfalls mit einem oder mehreren Substituenten $R^5$ substituiert ist, und wobei eine oder mehrere nicht benachbarte $CH_2$-Gruppen gegebenenfalls durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_6$-$C_{60}$-Aryl, das gegebenenfalls mit einem oder mehreren Substituenten $R^5$ substituiert ist; und $C_3$-$C_{57}$-Heteroaryl,
das gegebenenfalls mit einem oder mehreren Substituenten $R^5$ substituiert ist; und wobei davon abgesehen die Definitionen in Anspruch 1 gelten.

**5.** Organisches Molekül nach Anspruch 3 oder 4, wobei $R^b$ bei jedem Auftreten unabhängig ausgewählt ist aus der Gruppe bestehend aus

- H,
- Me, $^i$Pr, $^t$Bu, CN, $CF_3$,
- Ph, das gegebenenfalls mit einem oder mehreren Substituenten substituiert ist, die unabhängig voneinander ausgewählt sind aus der Gruppe bestehend aus Me, $^i$Pr, $^t$Bu, CN, $CF_3$ und Ph;
- Pyridinyl, das gegebenenfalls mit einem oder mehreren Substituenten substituiert ist, die unabhängig voneinander ausgewählt sind aus der Gruppe bestehend aus Me, $^i$Pr, $^t$Bu, CN, $CF_3$ und Ph;
- Pyrimidinyl, das gegebenenfalls mit einem oder mehreren Substituenten substituiert ist, die unabhängig voneinander ausgewählt sind aus der Gruppe bestehend aus Me, $^i$Pr, $^t$Bu, CN, $CF_3$ und Ph;
- Carbazolyl, das gegebenenfalls mit einem oder mehreren Substituenten substituiert ist, die unabhängig voneinander ausgewählt sind aus der Gruppe bestehend aus Me, $^i$Pr, $^t$Bu, CN, $CF_3$ und Ph;
- Triazinyl, das gegebenenfalls mit einem oder mehreren Substituenten substituiert ist, die unabhängig voneinander ausgewählt sind aus der Gruppe bestehend aus Me, $^i$Pr, $^t$Bu, CN, $CF_3$ und Ph;

und
- $N(Ph)_2$.

**6.** Verwendung eines organischen Moleküls nach einem oder mehreren der Ansprüche 1 bis 5 als ein Lumineszenzemitter und/oder als ein Wirtsmaterial und/oder als ein Elektronentransportmaterial und/oder als ein Lochinjektionsmaterial und/oder als ein Lochblockierungsmaterial in einer optoelektronischen Vorrichtung.

**7.** Verwendung nach Anspruch 6, wobei die optoelektronische Vorrichtung ausgewählt ist aus der Gruppe bestehend aus:

• organischen Leuchtdioden (OLEDs),
• lichtemittierenden elektrochemischen Zellen,
• OLED-Sensoren,
• organischen Dioden,
• organischen Solarzellen,
• organischen Transistoren,
• organischen Feldeffekt-Transistoren,
• organischen Lasern und
• Abwärtskonvertierungselementen.

**8.** Zusammensetzung, umfassend:

(a) ein organisches Molekül nach einem der Ansprüche 1 bis 5, insbesondere in Form eines Emitters und/oder

eines Wirts, und
(b) ein Emitter- und/oder Wirtsmaterial, das sich von dem organischen Molekül nach einem der Ansprüche 1 bis 5 unterscheidet, und
(c) gegebenenfalls einen Farbstoff und/oder ein Lösungsmittel.

**9.** Optoelektronische Vorrichtung, umfassend ein organisches Molekül nach einem oder mehreren der Ansprüche 1 bis 5 oder eine Zusammensetzung nach Anspruch 8, insbesondere in Form einer Vorrichtung, ausgewählt aus der Gruppe bestehend aus einer organischen Leuchtiode (OLED), einer lichtemittierenden elektrochemischen Zelle, einem OLED-Sensor, einer organischen Diode, einer organischen Solarzelle, einem organischen Transistor, einem organischen Feldeffekt-Transistor, einem organischen Laser und einem Abwärtskonvertierungselement.

**10.** Optoelektronische Vorrichtung nach Anspruch 9, umfassend:

- ein Substrat,
- eine Anode und
- eine Kathode, wobei die Anode oder die Kathode auf dem Substrat angeordnet sind, und
- eine lichtemittierende Schicht, die zwischen der Anode und der Kathode angeordnet ist und die das organische Molekül oder die Zusammensetzung umfasst.

**11.** Verfahren zum Herstellen einer optoelektronischen Vorrichtung, wobei ein organisches Molekül nach einem oder mehreren der Ansprüche 1 bis 5 oder eine Zusammensetzung nach Anspruch 8 verwendet wird.

**12.** Verfahren nach Anspruch 11, umfassend Verarbeiten des organischen Moleküls durch ein Vakuumverdampfungsverfahren oder aus einer Lösung.

## Revendications

**1.** Molécule organique comprenant :

- une première fraction chimique comprenant une structure de formule Iaa,

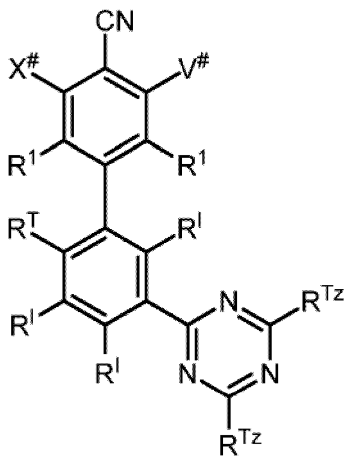

Formule Iaa

et
- trois secondes fractions chimiques, chacune comprenant indépendamment une structure de formule II,

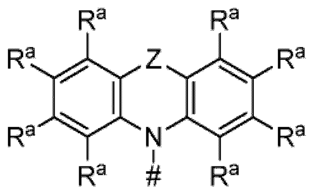

Formule II

dans laquelle la première fraction chimique est liée à chacune des trois secondes fractions chimiques via une liaison simple ;
dans laquelle
$X^{\#}$ est le site de liaison d'une liaison simple reliant la première fraction chimique à l'une des trois secondes fractions chimiques,
$V^{\#}$ est le site de liaison d'une liaison simple reliant la première fraction chimique à l'une des trois secondes fractions chimiques,
$R^T$ est le site de liaison d'une liaison simple reliant la première fraction chimique à l'une des trois secondes fractions chimiques ;
# représente le site de liaison d'une liaison simple reliant les secondes fractions chimiques à la première fraction chimique ;
Z est une liaison directe ; $R^1$ est, à chaque occurrence, indépendamment choisi dans le groupe consistant en : hydrogène, deutérium, alkyle en $C_1$ à $C_5$,
dans lequel un ou plusieurs atomes d'hydrogène sont facultativement substitués par du deutérium ; alcényle en $C_2$ à $C_8$, dans lequel un ou plusieurs atomes d'hydrogène sont facultativement substitués par du deutérium ; alcynyle en $C_2$ à $C_8$, dans lequel un ou plusieurs atomes d'hydrogène sont facultativement substitués par du deutérium ; et
aryle en $C_6$ à $C_{18}$, qui est facultativement substitué par un ou plusieurs substituants $R^6$; $R^I$ est, à chaque occurrence, indépendamment choisi dans le groupe consistant en : hydrogène, deutérium, alkyle en $C_1$ à $C_5$, dans lequel un ou plusieurs atomes d'hydrogène sont facultativement substitués par du deutérium ; alcényle en $C_2$ à $C_8$, dans lequel un ou plusieurs atomes d'hydrogène sont facultativement substitués par du deutérium ; alcynyle en $C_2$ à $C_8$, dans lequel un ou plusieurs atomes d'hydrogène sont facultativement substitués par du deutérium ; et
aryle en $C_6$ à $C_{18}$, qui est facultativement substitué par un ou plusieurs substituants $R^6$; $R^{Tz}$ est, à chaque occurrence, indépendamment choisi dans le groupe consistant en hydrogène, deutérium, alkyle en $C_1$ à $C_5$, dans lequel un ou plusieurs atomes d'hydrogène sont facultativement substitués par du deutérium ; aryle en $C_6$ à $C_{18}$, qui est facultativement substitué par un ou plusieurs substituants $R^6$; et $R^{6'}$ est, à chaque occurrence, indépendamment choisi dans le groupe consistant en :

hydrogène, deutérium, OPh, $CF_3$, CN, F, alkyle en $C_1$ à $C_5$,
dans lequel un ou plusieurs atomes d'hydrogène sont facultativement, indépendamment les uns des autres, substitués par du deutérium, CN, $CF_3$ ou F ; alcoxy en $C_1$ à $C_5$, dans lequel un ou plusieurs atomes d'hydrogène sont facultativement, indépendamment les uns des autres, substitués par du deutérium, CN, $CF_3$ ou F ;
thioalcoxy en $C_1$ à $C_5$, dans lequel un ou plusieurs atomes d'hydrogène sont facultativement, indépendamment les uns des autres, substitués par du deutérium, CN, $CF_3$ ou F ; alcényle en $C_2$ à $C_5$, dans lequel un ou plusieurs atomes d'hydrogène sont facultativement, indépendamment les uns des autres, substitués par du deutérium, CN, $CF_3$ ou F ; alcynyle en $C_2$ à $C_5$,
dans lequel un ou plusieurs atomes d'hydrogène sont facultativement, indépendamment les uns des autres, substitués par du deutérium, CN, $CF_3$ ou F ; aryle en $C_6$ à $C_{18}$, qui est facultativement substitué par un ou plusieurs substituants alkyle en $C_1$ à $C_5$ ; N(aryle en $C_6$ à $C_{18}$)$_2$ ; N(hétéroaryle en $C_3$ à $C_{17}$)$_2$, et N(hétéroaryl en $C_3$ à $C_{17}$)(aryle en $C_6$ à $C_{18}$) ;
$R^a$ est, à chaque occurrence, indépendamment choisi dans le groupe consistant en : hydrogène, deutérium, $N(R^5)_2$, $OR^5$, $Si(R^5)_3$, $B(OR^5)_2$, $OSO_2R^5$, $CF_3$, CN, F, Br, I, alkyle en $C_1$ à $C_{40}$, qui est facultativement substitué par un ou plusieurs substituants $R^5$ et dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et

dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ; thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ; alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ; alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$, qui est facultativement substitué par un ou plusieurs substituants $R^5$ ; et hétéroaryle en $C_3$ à $C_{57}$, qui est facultativement substitué par un ou plusieurs substituants $R^5$ ; $R^5$ est, à chaque occurrence, indépendamment choisi dans le groupe consistant en hydrogène, deutérium, $N(R^6)_2$, $OR^6$, $Si(R^6)_3$, $B(OR^6)_2$, $OSO_2R^6$, $CF_3$, CN, F, Br, I, alkyle en $C_1$ à $C_{40}$, qui est facultativement substitué par un ou plusieurs substituants $R^6$ et dans lequel un ou plusieurs groupes $CH_2$ non adjacents sont facultativement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcoxy en $C_1$ à $C_{40}$, qui est facultativement substitué par un ou plusieurs substituants $R^6$ et dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ; thioalcoxy en $C_1$ à $C_{40}$, qui est facultativement substitué par un ou plusieurs substituants $R^6$ et dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcényle en $C_2$ à $C_{40}$, qui est facultativement substitué par un ou plusieurs substituants $R^6$ et dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcynyle en $C_2$ à $C_{40}$, qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
aryle en $C_6$ à $C_{60}$, qui est facultativement substitué par un ou plusieurs substituants $R^6$ ; et hétéroaryle en $C_3$ à $C_{57}$, qui est facultativement substitué par un ou plusieurs substituants $R^6$ ; $R^6$ est, à chaque occurrence, indépendamment choisi dans le groupe consistant en : hydrogène, deutérium, OPh, $CF_3$, CN, F, alkyle en $C_1$ à $C_5$, dans lequel un ou plusieurs atomes d'hydrogène sont facultativement, indépendamment les uns des autres, substitués par du deutérium, CN, $CF_3$ ou F ; alcoxy en $C_1$ à $C_5$, dans lequel un ou plusieurs atomes d'hydrogène sont facultativement, indépendamment les uns des autres, substitués par du deutérium, CN, $CF_3$ ou F ; thioalcoxy en $C_1$ à $C_5$, dans lequel un ou plusieurs atomes d'hydrogène sont facultativement, indépendamment les uns des autres, substitués par du deutérium, CN, $CF_3$ ou F ; alcényle en $C_2$ à $C_5$, dans lequel un ou plusieurs atomes d'hydrogène sont facultativement, indépendamment les uns des autres, substitués par du deutérium, CN, $CF_3$ ou F ; alcynyle en $C_2$ à $C_5$, dans lequel un ou plusieurs atomes d'hydrogène sont facultativement, indépendamment les uns des autres, substitués par du deutérium, CN, $CF_3$ ou F ; aryle en $C_6$ à $C_{18}$, qui est facultativement substitué par un ou plusieurs substituants alkyle en $C_1$ à $C_5$ ; hétéroaryle en $C_3$ à $C_{17}$,
qui est facultativement substitué par un ou plusieurs substituants alkyle en $C_1$ à $C_5$ ; N(aryle en $C_6$ à $C_{18})_2$ ; N(hétéroaryle en $C_3$ à $C_{17})_2$, et N(hétéroaryl en $C_3$ à $C_{17}$)(aryle en $C_6$ à $C_{18}$) ; dans laquelle les substituants $R^a$ ou $R^5$ indépendamment les uns des autres forment facultativement un système de cycle mono- ou polycyclique, aliphatique, aromatique et/ou benzo-fusionné avec un ou plusieurs substituants $R^a$ ou $R^5$ ; dans laquelle les termes alkyle, alcényle, alcynyle, alcoxy et thioalcoxy peuvent faire référence à des substituants linéaires, ramifiés et cycliques.

**2.** Molécule organique selon la revendication 1, dans laquelle $R^I$ et $R^1$ sont indépendamment l'un de l'autre choisis dans le groupe consistant en hydrogène (H), méthyle, mésityle, tolyle et phényle.

**3.** Molécule organique selon une ou plusieurs des revendications 1 ou 2, dans laquelle les trois secondes fractions chimiques à chaque occurrence, indépendamment d'une autre, comprennent chacune une structure de formule IIb :

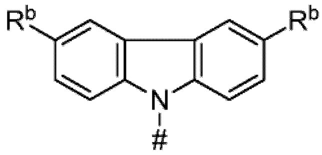

Formule IIb

dans laquelle

$R^b$ est, à chaque occurrence, indépendamment choisi dans le groupe consistant en : H, deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$, et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$, et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$, et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$, et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et hétéroaryle en $C_3$ à $C_{57}$,
qui est facultativement substitué par un ou plusieurs substituants $R^5$ ;
et dans laquelle, à part cela, les définitions de la revendication 1 s'appliquent.

**4.** Molécule organique selon une ou plusieurs des revendications 1 à 3, dans laquelle les trois secondes fractions chimiques à chaque occurrence, indépendamment d'une autre, comprennent chacune une structure de formule IIc :

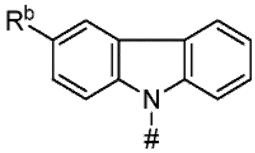

Formule IIc

dans laquelle

$R^b$ est, à chaque occurrence, indépendamment choisi dans le groupe consistant en H, deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$, et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,

qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et hétéroaryle en $C_3$ à $C_{57}$,
qui est facultativement substitué par un ou plusieurs substituants $R^5$ ;
et dans laquelle, à part cela, les définitions de la revendication 1 s'appliquent.

**5.** Molécule organique selon la revendication 3 ou 4, dans laquelle $R^b$ est, à chaque occurrence, indépendamment choisi dans le groupe consistant en

- H,
- Me, $^iPr$, $^tBu$, CN, $CF_3$,
- Ph, qui est facultativement substitué par un ou plusieurs substituants choisis, indépendamment les uns des autres, dans le groupe consistant en Me, $^iPr$, $^tBu$, CN, $CF_3$ et Ph ;
- pyridinyle, qui est facultativement substitué par un ou plusieurs substituants choisis, indépendamment les uns des autres, dans le groupe consistant en Me, $^iPr$, $^tBu$, CN, $CF_3$ et Ph ;
- pyrimidinyle, qui est facultativement substitué par un ou plusieurs substituants choisis, indépendamment les uns des autres, dans le groupe consistant en Me, $^iPr$, $^tBu$, CN, $CF_3$ et Ph ;
- carbazolyle, qui est facultativement substitué par un ou plusieurs substituants choisis, indépendamment les uns des autres, dans le groupe consistant en Me, $^iPr$, $^tBu$, CN, $CF_3$ et Ph ;
- triazinyle, qui est facultativement substitué par un ou plusieurs substituants choisis, indépendamment les uns des autres, dans le groupe consistant en Me, $^iPr$, $^tBu$, CN, $CF_3$ et Ph ;
et
- $N(Ph)_2$.

**6.** Utilisation d'une molécule organique selon une ou plusieurs des revendications 1 à 5, comme émetteur luminescent et/ou comme matériau hôte et/ou comme matériau de transport d'électrons et/ou comme matériau d'injection de trous et/ou comme matériau de blocage de trous dans un dispositif optoélectronique.

**7.** Utilisation selon la revendication 6, dans laquelle le dispositif optoélectronique est choisi dans le groupe consistant en :

• les diodes électroluminescentes organiques (OLED),
• les cellules électrochimiques électroluminescentes,
• les capteurs OLED,
• les diodes organiques,
• les cellules solaires organiques,
• les transistors organiques,
• les transistors à effet de champ organiques,
• les lasers organiques et
• les éléments de conversion vers le bas.

**8.** Composition, comprenant :

(a) une molécule organique selon une ou plusieurs des revendications 1 à 5, en particulier sous la forme d'un

émetteur et/ou d'un hôte, et
(b) un émetteur et/ou un matériau hôte, qui diffèrent de la molécule organique d'une ou plusieurs des revendications 1 à 5, et
(c) facultativement, une teinture et/ou un solvant.

**9.** Dispositif optoélectronique, comprenant une molécule organique selon une ou plusieurs des revendications 1 à 5 ou une composition selon la revendication 8, en particulier sous la forme d'un dispositif choisi dans le groupe consistant en une diode électroluminescente organique (OLED), une cellule électrochimique électroluminescente, un capteur OLED, une diode organique, une cellule solaire organique, un transistor organique, un transistor à effet de champ organique, un laser organique et un élément de conversion vers le bas.

**10.** Dispositif optoélectronique selon la revendication 9, comprenant :

- un substrat,
- une anode et
- une cathode, l'anode ou la cathode étant disposées sur le substrat, et
- une couche électroluminescente, qui est disposée entre l'anode et la cathode et qui comprend la molécule organique ou la composition.

**11.** Procédé de production d'un dispositif optoélectronique, dans lequel une molécule organique selon une ou plusieurs des revendications 1 à 5 ou une composition selon la revendication 8 est utilisée.

**12.** Procédé selon la revendication 11, comprenant le traitement de la molécule organique par un procédé d'évaporation sous vide ou à partir d'une solution.

Figure 1

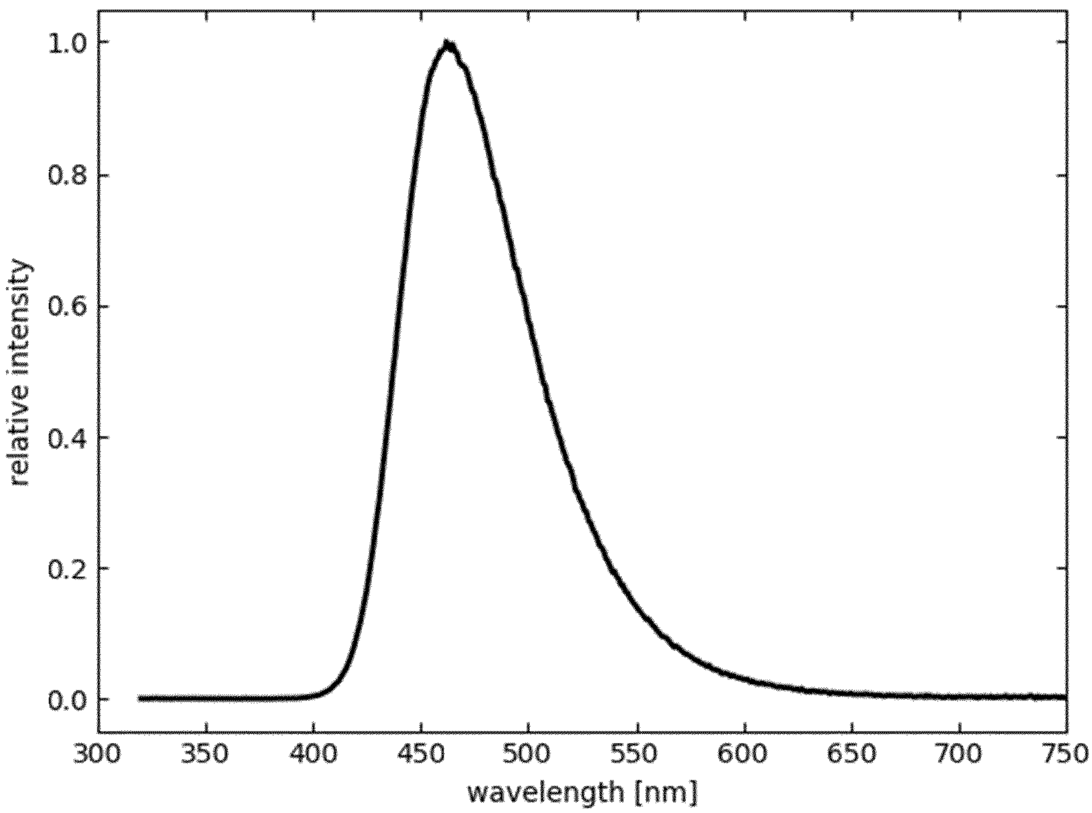
1.0
0.8
0.6
0.4
0.2
0.0
relative intensity
300
350
400
450
500
550
600
650
700
750
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3275968 A1
- KR 20160059733 A


### Non-patent literature cited in the description


- *CHEMICAL ABSTRACTS*, 2243446-04-8
- *CHEMICAL ABSTRACTS*, 1003298-73-4
- *CHEMICAL ABSTRACTS*, 51364-51-3
- *CHEMICAL ABSTRACTS*, 564483-18-7
- *CHEMICAL ABSTRACTS*, 73183-34-3